(19) Europäisches Patentamt
European Patent Office
Office européen des brevets

(11) Veröffentlichungsnummer: **0 344 634 B1**

(12) # EUROPÄISCHE PATENTSCHRIFT

(45) Veröffentlichungstag der Patentschrift: **30.03.94**

(51) Int. Cl.⁵: **C07D 209/34**, C07D 215/22, A61K 31/40, A61K 31/47

(21) Anmeldenummer: **89109511.9**

(22) Anmeldetag: **26.05.89**

(54) **Bicyclische Carboxamide, Verfahren zu ihrer Herstellung und diese Verbindungen enthaltende Arzneimittel.**

(30) Priorität: **03.06.88 DE 3818830**

(43) Veröffentlichungstag der Anmeldung:
**06.12.89 Patentblatt 89/49**

(45) Bekanntmachung des Hinweises auf die Patenterteilung:
**30.03.94 Patentblatt 94/13**

(84) Benannte Vertragsstaaten:
**AT BE CH DE ES FR GB GR IT LI LU NL SE**

(56) Entgegenhaltungen:
**EP-A- 0 236 140**
**EP-A- 0 255 134**
**DE-A- 3 204 892**

(73) Patentinhaber: **BOEHRINGER MANNHEIM GMBH**

**D-68298 Mannheim(DE)**

(72) Erfinder: **von der Saal, Wolfgang, Dr.rer.nat.**
**Neuer Burgweg 3**
**D-6940 Weinheim(DE)**
Erfinder: **Mertens, Alfred, Dr.rer.nat.**
**Beethovenstrasse 20**
**D-6905 Schriesheim(DE)**
Erfinder: **Boehm, Erwin, Dr.med.**
**Hinterer Rindweg 37**
**D-6802 Ladenburg(DE)**

**EP 0 344 634 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Verbindungen der allgemeinen Formel I

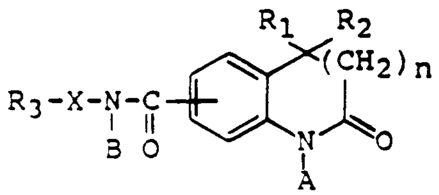

(I)

in der

A und B gleich oder verschieden sein können und jeweils ein Wasserstoffatom, eine $C_1$-$C_6$-Alkyl-, $C_2$-$C_6$-Alkenyl-, $C_2$-$C_6$-Alkinyl-, Benzyl- oder eine $C_3$-$C_7$-Cycloalkylgruppe bedeuten,

$R_1$ ein Wasserstoffatom, eine $C_1$-$C_6$-Alkyl-, $C_2$-$C_6$-Alkenyl-, oder $C_3$-$C_7$-Cycloalkylgruppe,

$R_2$ ein Wasserstoffatom, eine $C_1$-$C_6$-Alkyl-, $C_2$-$C_6$-Alkenyl-, $C_3$-$C_7$-Cycloalkyl-, $C_1$-$C_6$-Alkylcarbonyl-, $C_1$-$C_6$-Alkoxycarbonyl-, Aminocarbonyl- oder Hydrazinocarbonylgruppe bedeutet, oder $R_1$ und $R_2$ zusammen mit dem C-Atom, an das sie gebunden sind, einen $C_3$-$C_7$-Cycloalkylring bilden,

n die Werte 0 oder 1 annehmen kann,

X eine Bindung oder eine $C_1$-$C_6$-Alkylengruppe bedeutet,

$R_3$ einen aromatischen heterocyclischen Fünf- oder Sechsring mit 1 bis 4 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können und die Sechsringe gewünschtenfalls durch eine Pyridinyloxy- oder Phenyloxygruppe substituiert sein können oder die Fünf- oder Sechsringe mit einem Phenylring oder einem aromatischen Fünf- oder Sechsring mit 1 bis 4 Heteroatomen zu einem Bicyclus kondensiert sein können und gewünschtenfalls die Fünf- oder Sechsringe, die Bicyclen, die Pyridinyloxy- und die Phenyloxygruppe durch eine oder mehrere $C_1$-$C_6$-Alkyl-, $C_1$-$C_6$-Alkoxy-, $C_2$-$C_6$-Alkenyloxy-, $C_1$-$C_6$-Alkoxycarbonyl-, Carboxyl-, $C_1$-$C_6$-Alkylmercapto-, Hydroxy-, Nitro-, Amino-, Halogen- oder Cyangruppen substituiert sein können, oder

$R_3$ einen Phenylring der allgemeinen Formel II bedeutet,

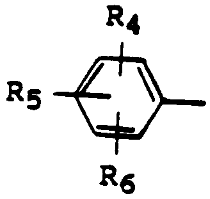

(II)

wobei $R_4$, $R_5$, $R_6$ gleich oder verschieden sein können und jeweils Wasserstoff, eine Imidazolylgruppe, eine gewünschtenfalls mit $C_1$-$C_6$-Alkylgruppen substituierte Oxopyridazinylgruppe, die gewünschtenfalls hydriert sein kann, eine $C_1$-$C_6$-Alkansulfonyloxy-, Trifluormethansulfonyloxy-, Phenylsulfonylamino-, $C_1$-$C_6$-Alkansulfonylamino-, Trifluormethansulfonylamino-, N-$C_1$-$C_6$-Alkylalkansulfonylamino-, N-$C_1$-$C_6$-Alkyltrifluormethansulfonylamino-, $C_1$-$C_6$-Alkylsulfenylmethyl-, $C_1$-$C_6$-Alkylsulfinylmethyl- oder $C_1$-$C_6$-Alkylsulfonylmethylgruppe, eine durch eine Hydroxy-, $C_1$-$C_6$-Alkoxy-, $C_1$-$C_6$-Alkylamino- oder Di-$C_1$-$C_6$-alkylaminogruppe substituierte Carbonylgruppe, eine durch eine Amino-, $C_1$-$C_6$-Alkylamino-, Di-$C_1$-$C_6$-alkylamino-, Piperidino- oder Morpholinogruppe substituierte Sulfonylgruppe, eine $C_1$-$C_6$-Alkylcarbonylamino-, Aminocarbonylamino- oder $C_1$-$C_6$-Alkylaminocarbonylaminogruppe, eine $C_1$-$C_6$-Alkylmermercapto-, $C_1$-$C_6$-Alkylsulfinyl- oder $C_1$-$C_6$-Alkylsulfonylgruppe, eine Nitro-, Halogen-, Amino-, Hydroxy-, $C_1$-$C_8$-Alkyl-, $C_1$-$C_6$-Alkoxy-, Pyridinyloxy-, $C_2$-$C_6$-Alkenyloxy-,$C_2$-$C_6$-Alkinyloxy-, Cyan-$C_1$-$C_6$-alkyloxy-, Carboxy-$C_1$-$C_6$-alkyloxy-, $C_1$-$C_6$-Alkoxycarbonyl-$C_1$-$C_6$-alkyloxy-, Di-$C_1$-$C_6$-alkylamino-, Trifluormethyl- oder Cyangruppe sein können

oder $R_3$ einen Naphthyl-, Tetrahydronaphthyl-, Biphenyl-, Methylendioxyphenyl- oder Ethylendioxyphenylrest bedeutet,

mit der Maßangabe, daß für den Fall, daß n die Zahl 1 und $R_1$ und $R_2$ ein Wasserstoffatom bedeuten, X keine $C_1$-$C_6$-Alkylengruppe sein kann,

deren Tautomere, optisch aktive Formen oder physiologisch verträgliche Salze anorganischer und organischer Säuren, Verfahren zu ihrer Herstellung und diese Verbindungen enthaltende Arzneimittel.

Für den Fall, daß Verbindungen der allgemeinen Formel I ein Asymmetriezentrum oder eine Asymmetrieebene besitzen, sind auch die optisch aktiven Formen und racemischen Gemische dieser Verbindungen Gegenstand der Erfindung.

Die Verbindungen der vorliegenden Erfindung weisen wertvolle pharmakologische Eigenschaften auf, insbesondere wirken sie hemmend auf die Erythrozytenaggregation und können somit Verwendung finden zur Behandlung von Krankheiten des Herz- und Kreislaufsystems, bei denen in der Pathogenese die Erythrozytenaggregation eine wichtige Rolle spielt, wie z.B. periphere, coronare und cerebrale Durchblutungsstörungen und Schockzustände. Die Verbindungen beeinflussen darüber hinaus auch die Thrombozytenfunktion. Sie wirken throbozytenaggregationshemmend und blutdrucksenkend und können ferner die Herzkraft steigern.

Aus dem Stand der Technik sind Verbindungen mit ähnlicher Struktur wie die der vorliegenden Erfindung bereits bekannt:

a) In der Patentschrift DE 3204892 (Anmeldetag 12.2.82, Otsuka) sind Verbindungen (Carbostyrilderivate), beschrieben, in denen A ein Wasserstoffatom, eine Alkyl-, Alkenyl-, Alkinyl- oder Phenylalkylgruppe, $R_1$ und $R_2$ ein Wasserstoffatom, n die Zahl 1, B eine Alkylgruppe, X eine Alkylengruppe und $R_3$ eine Phenylgruppe, die durch eine Alkoxy-, Halogen oder Alkylendioxygruppe substituiert sein kann, darstellen. Diese Verbindungen erhöhen die myokardiale Kontraktion, den Koronarblutfluß und haben eine blutdrucksenkende Wirkung, und werden deshalb als Cardiotonika eingesetzt.

b) In der Patentschrift DE 3204892 wird unter anderem auch auf Verbindungen (Carbostyrilderivate) verwiesen, wobei A und B die unter a) angegebenen Bedeutungen haben, und sowohl $R_1$ als auch $R_2$ jeweils ein Wasserstoffatom bedeuten, X eine Methylengruppe und $R_3$ eine gewünschtenfalls substituierte Phenylgruppe darstellen. Diese Verbindungen sind lediglich als Zwischenprodukte für die Herstellung von pharmazeutischen Chemikalien beschrieben.

Die aus dem Stand der Technik bekannten Verbindungen werden für den Fall, daß n die Zahl 1 bedeutet, $R_1$ und $R_2$ ein Wasserstoffatom darstellen und X dann keine Alkylengruppe bedeuten darf, von der vorliegenden Erfindung nicht umfaßt.

Bedeutet in der allgemeinen Formel I A oder B eine Alkylgruppe, so sind darunter geradkettige oder verzweigte Gruppen mit 1-6 C-Atomen zu verstehen. In diesem Sinne kommen insbesondere die Methyl-, Ethyl-, Propyl-, Butyl-, Isopropyl-, Isobutyl- und tert.-Butylgruppen in Frage. Bedeutet in der allgemeinen Formel I A oder B eine Alkenyl- oder Alkinylgruppe, so sind darunter geradkettige oder verzweigte Gruppen mit 2-6 C-Atomen zu verstehen. In diesem Sinne kommen insbesondere die Allyl-, Propargyl-, Butenyl- und Isobutenylgruppen in Frage. Bedeutet in der allgemeinen Formel I A oder B eine Cycloalkylgruppe, so sind darunter Ringe mit 3-7 C-Atomen zu verstehen. In diesem Sinne kommen insbesondere die Cyclopropyl-, Cyclobutyl-, Cyclopentyl- und Cyclohexylgruppen in Frage.

Für den Fall, daß $R_1$ ein Wasserstoffatom, eine Alkyl- oder Alkenylgruppe bedeutet, können $R_1$ und $R_2$ gleich oder verschieden sein. $R_2$ kann ferner eine durch eine Alkyl-, Alkoxy-, Amino- oder Hydrazinogruppe substituierte Carbonylgruppe darstellen. Die zuvor bei $R_1$ und $R_2$ genannten Alkyl-oder Alkoxyteile können geradkettig oder verzweigt, gesättigt oder ungesättigt sein und 1 bis 6 bzw. 2 bis 6 Kohlenstoffatome enthalten. Insbesondere kommen jedoch Wasserstoff, die Methyl-, Ethyl-, Allyl-, Acetyl-, Propionyl-, Methoxycarbonyl-, Ethoxycarbonyl- und Hydrazinocarbonylgruppe für $R_1$ bzw. $R_2$ in Frage.

Für den Fall, daß $R_1$ ein Wasserstoffatom darstellt, so ist $R_2$ bevorzugt eine geradkettige oder verzweigte Alkylgruppe mit 1 bis 6 Kohlenstoffatomen oder eine durch eine Alkyl-, Alkoxy-, Amino- oder Hydrazinogruppe substituierte Carbonylgruppe, Bevorzugt in diesem Sinne sind die Methyl-, Ethyl-, Isopropyl-, Isobutyl-, Pentyl-, Allyl-, Acetyl-, Propionyl-, Methoxycarbonyl-, Ethoxycarbonyl-, und Hydrazinocarbonylgruppe.

$R_1$ und $R_2$ können zusammen mit dem C-Atom, an das sie gebunden sind, auch einen Cycloalkylring mit 3 bis 8 Kohlenstoffatomen bilden, vorzugsweise handelt es sich dabei um die Spirocyclopropyl-, Spirocyclobutyl-, Spirocyclopentyl-, und Spirocyclohexylgruppe.

Stellt $R_1$ eine Cycloalkylgruppe dar, so kann diese 3-7 Kohlenstoffatome enthalten. Bevorzugt kommen die bei der Definition von A oder B genannten Gruppen in Frage, insbesondere jedoch der Cyclopentyl- und Cyclohexylrest.

Bedeutet in der allgemeinen Formel I X eine Alkylengruppe, so sind darunter geradkettige oder verzweigte Ketten mit 1 bis 6 C-Atomen zu verstehen. Besonders bevorzugt sind die Methylen-, Ethylen-, Propylen- und Butylengruppe.

Nimmt in der allgemeinen Formel I n den Wert 0 an, so handelt es sich bei den Verbindungen um Substituierte
2,3-Dihydro-2-oxo-1H-indol-4-carboxamide oder 2,3-Dihydro-2-oxo-1H-indol-5-carboxamide oder
2,3-Dihydro-2-oxo-1H-indol-6-carboxamide oder
2,3-Dihydro-2-oxo-1H-indol-7-carboxamide; nimmt n den Wert 1 an, so werden die Substanzen als
1,2,3,4-Tetrahydro-2-oxo-5-chinolincarboxamide oder
1,2,3,4-Tetrahydro-2-oxo-6-chinolincarboxamide oder
1,2,3,4-Tetrahydro-2-oxo-7-chinolincarboxamide oder
1,2,3,4-Tetrahydro-2-oxo-8-chinolincarboxamide bezeichnet.

Die bei $R_3$ angegebenen Heterocyclischen Fünf- oder Sechsringe mit 1 bis 4 bzw. 1 bis 5 Heteroatomen, wobei die Heteroatome der vorgenannten Fünf- oder Sechsringe gleich oder verschieden sein können und Stickstoff, Sauerstoff oder Schwefel bedeuten und gegebenenfalls an einem oder mehreren Stickstoffatomen ein Sauerstoff tragen können, bedeuten bevorzugt den Pyrrolyl-, Furanyl-, Thiophenyl-, Pyrazolyl-, Imidazolyl-, Thiazolyl-, Tetrazolyl-, Isothiazolyl-, Oxazolyl-, Isoxazolyl-, Triazolyl-, Thiadiazolyl-, Oxadiazolyl-, Pyrazinyl-, N,N′-Dioxy-pyrazinyl, Pyrimidinyl-, N,N′-Dioxypyrimidinyl-, Pyridazinyl-, Oxazinyl-, Thiazinyl-, Triazinyl-, Tetrazinyl-, Pyridinyl- und den N-Oxypyridinylrest.

Sind die aromatischen heterocyclischen Fünf- oder Sechsringe mit einem Phenylring kondensiert, so sind der Indolyl-, Indazolyl-, Benzimidazolyl-, Chinolinyl-, Isochinolinyl-, Cinnolinyl-, Phthalazinyl-, Chinazolinyl-, Chinoxalinyl-, Benzofuranyl-, Benzothiophenyl-, Benzoxazolyl-, Benzoisoxazolyl-, Benzothiazolyl-, Benzoisothiazolyl-, Benzotriazolyl- und der Benzothiadiazolylrest bevorzugt.

Sind die aromatischen heterocyclischen Fünf- oder Sechsringe mit einem weiteren aromatischen heterocyclischen Fünf- oder Sechsring zu einem Bicyclus kondensiert, so sind darunter bevorzugt der Naphthyridinyl-, Pteridinyl-, Purinyl-, Indolizinyl-, Thiopheno[2,3-b]pyrazinyl-, Imidazo[1,2-a]pyridinyl- und der Triazolo[4,3-a]pyridinylrest.

Besonders bevorzugt für $R_3$ kommen der Pyridinyl-, Tetrazolyl-, Triazolyl-, 1,2,4-Triazolo[4,3-a]pyridinyl-, Methylendioxyphenyl-, Ethylendioxyphenyl-, Naphthyl-, Tetrahydronaphthyl-, Chinolinyl-, Biphenyl- und Phenylring der allgemeinen Formel II in Frage.

Sind die vorher genannten Sechsringe durch einen Pyridinyloxyrest substituiert, so ist es bevorzugt der 3-Pyridinyloxyrest. Sind die vorgenannten Sechsringe durch einen Phenyloxyrest substituiert, so ist besonders bevorzugt eine Verknüpfung mit einer Pyridinylgruppe als heterocyclischem Sechsring.

Alkyl-, Alkoxy- und Alkylmercapto-Substituenten in den heterocyclischen Fünf- oder Sechsringen, den Bicyclen, der Pyridinyloxy- oder Phenyloxygruppe können 1 bis 6, vorzugsweise 1 bis 4 Kohelenstoffatome enthalten. Bevorzugt ist der Methyl-, Ethyl-, Methoxy-, Ethoxy-, Methylmercapto- und Ethylmercaptorest. Unter Halogen-Substituenten sind Fluor, Chlor und Brom, vorzugsweise Fluor und Chlor zu verstehen.

Bedeutet $R_3$ einen Phenylring der allgemeinen Formel II, so kann der Alkylteil der bei $R_4$, $R_5$ und $R_6$ genannten Substituenten 1 bis 8 Kohlenstoffatome enthalten, vorzugsweise 1 bis 5 Kohlenstoffatome. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, N-Methyl-methansulfonylamino-, N-Ethyl-methansulfonylamino-, N-Methyl-ethansulfonylamino-, N-Ethyl-ethansulfonylamino-, N-Isopropyl-ethansulfonylamino-, N-Methyl-n-propansulfonylamino-, N-n-Propyl-n-propansulfonylamino-,N-Methyl-trifluormethansulfonylamino-, N-Ethyltrifluormethansulfonylamino-,N-Iso-propyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl-, Di-n-propyl-aminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methyl-isopropylaminosulfonyl-, Propionylamino-, Methylcarbonylamino-, Ethylaminocarbonylamino- oder Propylaminocarbonylaminogruppe, eine Methyl-, Ethyl-, Propyl-, Butyl-, Pentyl-, Hexyl-, Heptyl-, Octyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonyl-methyloxy-, Methoxycarbonylethyloxy-, Methylmercapto-, Ethyl-mercapto-, Methylsulfinyl-, Ethylsulfinyl-, Methylsulfonyl-oder Ethylsulfonylgruppe.

Insbesondere sind bevorzugt für $R_4$ Wasserstoff, eine 1-Imidazolyl-, 2-Imidazolyl-, 6-Oxo-(1H)-3-pyridazinyl-, 4,5-Dihydro-6-oxo-(1H)-3-pyridazinyl-, 4,5-Dihydro-4-methyl-6-oxo-(1H)-3-pyridazinylgruppe, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1 bis 4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe, vorzugsweise mit 1 bis 3 Kohlenstoffatomen, die 3-Pyridinyloxygruppe, eine Cyanmethyloxy-, Methoxycarbonylmethyloxygruppe oder die Trifluormethylgruppe,
für $R_5$ Wasserstoff, eine Alkylgruppe mit 1 bis 3 Kohlenstoffatomen, eine Alkoxy- oder Dialkylaminogruppe mit 1 oder 2 Kohlenstoffatomen in jedem Alkylteil, ein Halogenatom oder eine Aminogruppe und
für $R_6$ Wasserstoff oder die Methoxygruppe.

Der Phenylteil kann 1 bis 3 der genannten Substituenten tragen.

Bevorzugte monosubstituierte Phenylverbindungen sind die Hydroxy-, $C_1$-$C_8$ Alkyl-, $C_1$-$C_3$ Alkoxy-, Allyloxy-, Propargyloxy-, Cyanmethyloxy-, Methoxycarbonylmethyloxy-, Halogen-, Nitro-, Cyan-, Aminocarbonyl-, Amino-, $C_1$-$C_3$ Dialkylamino-, $C_1$-$C_3$ Alkylmercapto-, $C_1$-$C_3$ Alkylsulfinyl-, $C_1$-$C_3$ Alkylsulfonyl-, $C_1$-$C_3$ Alkylsulfonyloxy-, 3-Pyridinyloxy- und 4,5-Dihydro-6-oxo-(1H)-pyridazinyl-phenyle, wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonyl-amino- oder N-Alkyl-trifluormethyl-sulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, eine Alkylaminosulfonyl-, Alkylcarbonylamino-, Aminocarbonylamino- oder N-Alkyl-aminocarbonylaminogruppe,eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl- oder Alkylsulfonylgruppe, wobei die beiden Substituenten gleich oder verschieden sein können und in 2,3-, 2,4-, 2,5-, 2,6-, 3,4- und 3,5-Stellung, bevorzugt jedoch in 2,4-, 2,5- und 3,4-Stellung stehen können und die vorgenannten Alkylreste, allein oder in Kombination mit anderen Resten, 1 bis 3 C-Atome aufweisen können.

Bevorzugter trisubstituierter Phenylrest ist der 3,4,5-Trimethoxyphenylrest.

Besonders bevorzugte Verbindungen der allgemeinen Formel I sind Verbindungen, in denen

A und B unabhängig voneinander ein Wasserstoffatom, eine Benzyl-, eine $C_1$-$C_4$-Alkyl-, insbesondere eine Methyl-, Ethyl-, Propyl-, Isopropyl- oder Isobutyl-; oder eine $C_2$-$C_4$-Alkenyl-, insbesondere eine Allylgruppe bedeutet,

$R_1$ und $R_2$ gleich sind und $C_1$-$C_4$-Alkylgruppen, insbesondere Methylgruppen bedeuten oder $R_1$ und $R_2$ zusammen mit dem Kohlenstoffatom, an das sie gebunden sind, einen $C_5$-$C_6$-Cycloalkylring, insbesondere einen Cyclopentylring bilden,

X eine Bindung oder eine $C_1$-$C_4$-Alkylenkette, insbesondere die Ethylenkette darstellt und

$R_3$ den Pyrrolyl-, Furanyl-, Thiophenyl-, Pyrazolyl-,Imidazolyl-, Isothiazolyl-, Thiazolyl-, Oxazolyl-, Triazolyl-, Tetrazolyl-, Thiadiazolyl-, Isoxazolyl, Oxadiazolyl-, Pyridinyl-, N-Oxypyridinyl-, Pyrazinyl-, N,N′-Dioxypyrazinyl-,Pyrimidinyl-, N,N′-Dioxypyrimidinyl-, Pyridazinyl-, Oxazinyl-, Thiazinyl-, Triazinyl-, Tetrazinyl-, Indolyl-, Benzimidazolyl-, Benzthiazolyl-, Indazolyl-, Chinolinyl-, Pyridinyloxy-pyridinyl- oder Phenoxypyridinyl-rest darstellt sowie deren durch $C_1$-$C_4$-Alkyl-, insbesondere Methyl-, Ethyl-; $C_1$-$C_4$-Alkoxy, insbesondere Methoxy-, Ethoxy-; $C_2$-$C_4$-Alkenyloxy-, insbesondere Allyloxy-, Butenyloxy-; Methylendioxy-; $C_1$-$C_4$-Alkylmercapto-,insbesondere Methylmercapto-; Chlor-; Amino- oder Hydroxy substituierten Derivate oder in der

$R_3$ den Phenylrest der allgemeinen Formel II bedeutet, in der

$R_4$ ein Wasserstoffatom, die Methansulfonyloxy-, Trifluormethansulfonyloxy-, Methansulfonylamino-, Trifluormethansulfonylamino-, Methylmercapto-, Methylsulfinyl-, Methylsulfonyl-, Hydroxy-, Methyl-, Methoxy-, Propargyloxy-, Trifluormethyl-, Imidazolyl-, 3-Pyridinyloxygruppe-, Pyridazinyl-, wie z.B. 4,5-Dihydro-6-oxo-(1H)-pyridazinyl-oder 4,5-Dihydro-4-methyl-6-oxo-(1H)-pyridazinylgruppe, eine Allyloxy- oder Isobutenyloxygruppe, oder eine Ethoxycarbonylmethyloxygruppe, eine Chlor-, Fluor-, Cyano-, Dimethylamino- oder Diethylaminogruppe,

$R_5$ Wasserstoff, die Methyl-, Methoxy-, Dimethylamino- oder Chlorgruppe,

$R_6$ Wasserstoff oder die Methoxygruppe bedeutet, oder

$R_3$ einen Naphthyl-, Tetrahydronaphthyl-, Biphenyl-, Methylendioxyphenyl- oder Ethylendioxyphenylrest

bedeutet.

Die Verknüpfung des Amidrestes mit dem Bicyclus erfolgt vorzugsweise derart, daß man für n = 0 die Substanzen als substituierte 2,3-Dihydro-2-oxo-1H-indol-5-carboxamide oder 2,3-Dihydro-2-oxo-1H-indol-6-carboxamide bezeichnet und für n = 1 als 1,2,3,4-Tetrahydro-2-oxo-6-chinolincarboxamide oder 1,2,3,4-Tetrahydro-2-oxo-7-chinolincarboxamide bezeichnet.

Insbesondere sind Verbindungen der Formel I bevorzugt, wobei im Fall n = 0, die $R_3$-X-N(B)-CO-Gruppe in 5- oder 6-Stellung des Oxindolrings steht, und im Fall n = 1, die $R_3$-X-N(B)-CO-Gruppe in 7- oder 8-Stellung des Tetrahydrochinolinonrings steht.

Ferner sind solche Verbindungen der Formel I bevorzugt, in der A ein Wasserstoffatom, eine Benzyl-, $C_1$-$C_6$-Alkyl- oder $C_2$- $C_4$-Alkenylgruppe und B ein Wasserstoffatom oder ein $C_1$-$C_6$-Alkylgruppe bedeutet.

Bevorzugt sind ferner Verbindungen der Formel I, in der $R_3$ einen Phenylrest bedeutet, der unsubstituiert oder durch $C_1$-$C_4$-Alkylsulfonyloxy-, Trifluormethansulfonyloxy-, $C_1$-$C_4$-Alkyl-sulfonylamino-, Trifluormethansulfonylamino-, $C_1$-$C_4$-Alkylmercapto-,$C_1$-$C_4$-Alkylsulfinyl-, $C_1$-$C_4$-Alkylsulfonyl-, Hydroxy-, $C_1$-$C_8$-Alkyl-, $C_1$-$C_4$-Alkoxy-, $C_2$-$C_4$-Alkinyloxy-, Trifluormethyl-, Imidazolyl-, Pyridinyloxy-, 4,5-dihydro-4-$C_1$-$C_4$-Alkyl-6-oxo-(1H)-Pyridazinyl-, $C_2$-$C_4$-Alkenyloxy-, $C_1$-$C_4$-Alkoxycarbonyl-$C_1$-$C_4$-alkyloxy-, Nitro-, Amino-, $C_1$-$C_4$-Alkylcarbonylamino-, $C_1$-$C_4$-Alkoxycarbonyl-, Chlor-, Fluor-, Cyano- oder Di-$C_1$-$C_4$-Alkylaminogruppen substituiert sein kann, oder $R_3$ einen Methylendioxyphenyl-, Ethylendioxyphenyl- Naphthyl-, Tetrahydronaphthyl- oder Biphenylrest bedeutet.

Bevorzugte Verbindungen der Formel I sind ferner Derivate, wobei $R_1$ und $R_2$ jeweils eine Methylgruppe darstellen, und A und B ein Wasserstoffatom und X einen Valenzstrich bedeuten.

Die Verbindungen der allgemeinen Formel I können nach an sich bekannten Methoden hergestellt werden. Besonders vorteilhaft sind die folgenden Verfahren.

Verbindungen der allgemeinen Formel I stellt man her, indem man ein Amin der allgemeinen Formel III

$R_3$-X-NH-B (III),

in der $R_3$, X und B die oben angegebenen Bedeutungen haben, mit einer Carbonsäure der allgemeinen Formel IV

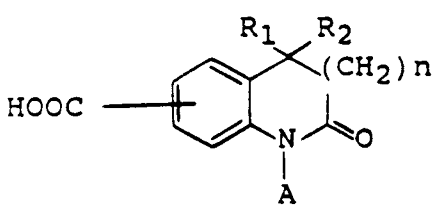

(IV)

in der A, $R_1$, $R_2$ und n die oben angegebenen Bedeutungen haben, oder mit einem reaktiven Derivat hiervon, acyliert. Unter reaktiven Derivaten versteht man Ester wie Methyl- oder Ethylester, Anhydride und Säurehalogenide wie Säurechloride oder Bromide.

Die Bevorzugte Methode zur Umsetzung mit den Aminen der allgemeinen Formel III besteht in der Reaktion von etwa äquimolaren Mengen des Amins und der Säure in Gegenwart eines wasserentziehenden Mittels. Dafür kommt beispielsweise Polyphosphorsäure in Betracht, die dann gleichzeitig als Lösungsmittel dient. Die Reaktionen laufen zwischen 50 °C und 200 °C ab. Die Endprodukte der allgemeinen Formel I fallen im allgemeinen nach Zugabe von Wasser aus und werden nach Filtration durch Umkristallisation oder säulenchromatographisch gereinigt.

Eine weitere bevorzugte Methode zur Herstellung von Verbindungen der allgemeinen Formel I besteht in der Umsetzung von etwa äquimolaren Mengen des Amins III und der Säure IV in einem geeig-neten Lösungsmittel mit etwa einer äquivalenten Menge eines Halogenierungsmittels, wie Phosphortrichlorid, Phosphorpentachlorid oder Thionylchlorid, bei Temperaturen zwischen Raumtemperatur und der Rückflußtemperatur der Mischung. Geeignete Lösungsmittel sind Methylenchlorid, Tetrachlorkohlenstoff, Diethylether, Toluol, Xylol oder Chlorbenzol. Im allgemeinen fällt das Produkt aus der Lösung aus und wird durch Filtration gewonnen. Falls erforderlich, kann die Reaktionsmischung konzentriert werden bis zu einem Punkt, bei dem das Produkt aus der Lösung auszufallen beginnt. Als weitere Kondensationsmittel bei dieser Reaktion kommen saure Kationenaustauscher, Sulfoniumsalze, Schwefelsäurehalogenide, 2-Halogenpyridiniumsalze, Phosphoniumsalze und N,N′-Dicyclohexyl-carbodiimid in Betracht.

Setzt man anstelle der Carbonsäuren ihre Ester ein, so arbeitet man in Gegenwart oder Abwesenheit spezieller Lösungsmittel bei Temperaturen im Bereich von 20 °C bis zur Siedehitze des Gemisches. Bevorzugt ist dabei die Reaktion etwa äquimolarer Mengen des Amins und des Esters in Polyphosphorsäure bei 50 °C bis 200 °C, jedoch kann man auch in einem inerten Lösungsmittel wie Methylenchlorid, Benzol, Toluol, Chlorbenzol am besten in Gegenwart von etwas mehr als einem Äquivalent einer Base wie Natriumethanolat oder Butyllithium oder von Natriumhydrid in Dimethylsulfoxid arbeiten.

Setzt man anstelle der Carbonsäure IV ihre Anhydride ein, so kann man die Umsetzung mit den Aminen der allgemeinen Formel III schon bei etwas niedrigeren Temperaturen vornehmen. Bevorzugt arbeitet man in einem inerten Lösungsmittel wie Dichlormethan, Diethylether, Benzol, Toluol, bei Temperaturen zwischen Raumtemperatur und 60 °C. Man gibt dabei das Amin und das Anhydrid in etwa äquimolaren Mengen zusammen, wobei im allgemeinen eine exotherme Reaktion einsetzt. Nach Abklingen wird zur Vervollständigung der Reaktion noch einige Zeit gelinde erwärmt.

Setzt man anstelle der Carbonsäure ein Säurehalogenid ein, so arbeitet man am besten bei Temperaturen zwischen - 10 °C und Raumtemperatur. Bevorzugt geht man dabei so vor, daß nach Schotten-Baumann zur wässrigen Lösung des Amins der allgemeinen Formel III, welche noch eine Base wie Alkalihydroxid, Natriumcarbonat oder Pyridin enthält, das Säurechlorid unter Eiskühlung langsam zutropft und anschließend noch einige Zeit bei Raumtemperatur stehen läßt. Diese Reaktion ist nicht nur in Wasser möglich, sondern auch in organischen Lösungsmitteln wie Methylenchlorid, Ether, Benzol oder Toluol. Auch ohne säurebindende Mittel lassen sich die Amine durch Carbonsäurechloride nahezu quantitativ acylieren, indem man das Amin und das Carbonsäurechlorid in einem inerten Lösungsmittel wie Methylenchlorid, Benzol oder Toluol bis zur Beendigung der Gasentwicklung kocht, was 1 bis 24 Stunden dauert. Gibt man jedoch ein säurebindendes Mittel wie Triethylamin oder Pyridin in geringem Überschuß zu, so läuft die Reaktion schon bei Temperaturen zwischen - 10 °C und Raumtemperatur ab.

Die Verbindungen der allgemeinen Formel III sind literaturbekannt. Von den Verbindungen der allgemeinen Formel IV ist die Herstellung der 2,3-Dihydro-2-oxo-1H-indol-4-carbonsäure beschrieben in J. von Braun, G. Hahn, Chem.Ber. 56 (1923) 2342, die Herstellung der 2,3-Dihydro-2-oxo-1H-indol-5-carbonsäure in EP 168003 (15.1.1986, Otsuka), die Herstellung der 1-Ethyl-2,3-dihydro-2-oxo-(1H)-indol-5-carbonsäure in EP 181 136 (14.5.86, Pfizer), die Herstellung der 1′,2′-Dihydro-2′-oxo-spiro[cyclopropan-1,3′-(3H)-indol]-6′-carbonsäure in JP 57/102863 (26.6.82, Takeda), die Herstellung der 2,3-Dihydro-2-oxo-(1H)-indol-7-carbonsäure in US 3631177 (28.12.1971, SK&F), die Herstellung der 2,3-Dihydro-3,3-dimethyl-2-oxo-1H-indol-5-carbonsäure in R.F. Moore, S.G.P. Plant, J. Chem.Soc. 1951 3475, die Herstellung der 2,3-Dihydro-2-oxo-1H-indol-6-carbonsäure in M. Fileti, E. Cairola, J.prakt.Chem. 46 (1982) 563, die Herstellung der 4-Methyl-1,2,3,4-Tetrahydro-2-oxo-6-chinolincarboxamide in JP 63,112584 (17.5.1988, Yoshitomi). Die anderen Verbindungen der allgemeinen Formel IV sind neu und ebenfalls Gegenstand der Erfindung.

Das in den letztgenannten Literaturstellen angewendete Verfahren zur Herstellung der namentlich genannten Verbindungen läßt sich auch auf die neuen Verbindungen der allgemeinen Formel IV anwenden. Es besteht in der Reduktion von Verbindungen der allgemeinen Formel V

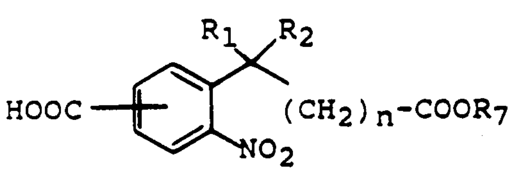

(V)

in der $R_1$, $R_2$ und n die oben genannten Bedeutungen besitzten und $R_7$ ein Wasserstoffatom oder eine Alkylgruppe, vorzugsweise eine Methyl- oder Ethylgruppe darstellt. Die Reduktion verläuft unter Ringschluß, so daß man unmittelbar die Verbindungen der allgemeinen Formel IV erhält. Die Reduktion wird vorzugsweise in einem Lösungsmittel wie Wasser, Methanol, Ethanol, Eisessig, Essigsäureethylester, DMF oder einem Gemisch dieser Lösungsmittel, mit Wasserstoff in Gegenwart eines Katalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit mit Hydrazin in Gegenwart von Raney-Nickel bei Temperaturen zwischen 0 und 100 °C, vorzugsweise jedoch bei Raumtemperatur, durchgeführt.

Bei dieser Reduktion der Verbindungen der allgemeinen Formel V entstehen zunächst die Verbindungen der allgemeinen Formel IV, in der A ein Wasserstoffatom bedeutet, welche dann gewünschtenfalls alkyliert werden können zu den Verbindungen der allgemeinen formel IV, in denen A eine Alkyl-, Alkenyl-,

Alkinyl-oder Cycloalkylgruppe bedeutet. Diese Alkylierungen werden vorzugsweise in einem Lösungsmittel wie Aceton, Methylethylketon, Ether, Benzol, Toluol oder Dimethylformamid bei Temperaturen zwischen -30 °C und +100 °C, vorzugsweise bei 0-80°C in Gegenwart einer Base, wie Kaliumhydroxid oder Natriumcarbonat und eines Alkylierungsmittels wie Alkylhalogeniden, Alkenylhalogeniden, Alkinylhalogeniden, Cycloalkylhalogeniden oder den entsprechenden -sulfaten durchgeführt.

Die Verbindungen der allgemeinen Formel V stellt man durch Nitrieren von Verbindungen der allgemeinen Formel VI her,

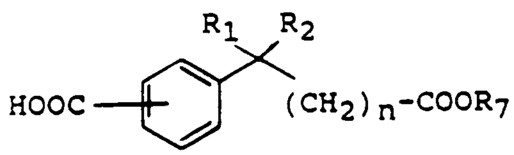

(VI)

in der $R_1$, $R_2$, $R_7$ und n die oben angegebenen Bedeutungen haben. Die Nitrierung führt man bevorzugt mit Salpetersäure in Schwefelsäure bei Temperaturen zwischen - 20 °C und + 50 °C durch. Sie kann jedoch auch ohne Schwefelsäure oder an deren Stelle in Wasser, Eisessig oder Acetanhydrid durchgeführt werden, oder mit $N_2O_5$ in $CCl_4$ in Gegenwart von $P_2O_5$. Als Nitrierungsreagenzien können auch Anhydride wie Acetylnitrat oder Nitrylhalogenide mit $FeCl_3$, Methylnitrat und $BF_3$ oder Nitroniumsalze wie $NO_2BF_4$, $NO_2PF_6$ oder $NO_2CF_3SO_3$ dienen. Zur Nitrierung kann auch eine Mischung als Salpetersäure und salpetriger Säure benutzt werden, welche $N_2O_4$ als eigentliche nitrierende Spezies liefert.

Ein weiteres Verfahren zur Herstellung von Carbonsäuren der allgemeinen Formel IV besteht in der Verseifung der Nitrile der allgemeinen Formel VII

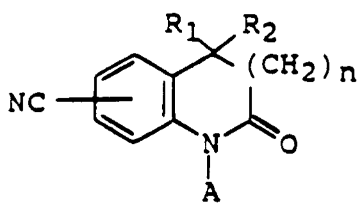

(VII)

in der A, $R_1$, $R_2$ und n die oben angegebenen Bedeutungen haben. Die Nitrile der allgemeinen Formel VII erhält man aus den Aminen der allgemeinen Formel VIII

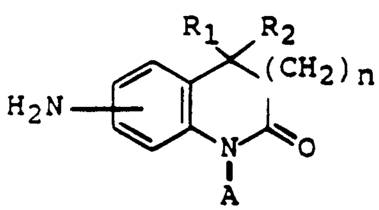

(VIII)

in der A, $R_1$, $R_2$ und n die oben angegebenen Bedeutungen haben, durch Diazotierung und Umsetzung mit Cyanid (Sandmeyer-Reaktion). Die Verbindungen der allgemeinen Formel VIII sind aus der deutschen Patentanmeldung Az. 38 03 775.0 bzw. der europäischen Patentanmeldung Az. 89 101 868.1 (Boehringer Mannheim) und EP-OS 161,632 bekannt.

Von den Verbindungen der allgemeinen Formel VII ist die Synthese von 2,3-Dihydro-2-oxo-(1H)-indol-5-carbonitril beschrieben in G.P. Gassmann, D.P. Gilbert und T. -Y. Luh, J.Org.Chem. 42 (1977) 1340. Die anderen Verbindungen der allgemeinen Formel VII sind neu und ebenfalls Gegenstand der Erfindung.

Die Diazotierung von Verbindungen der allgemeinen Formel VIII führt man vorzugsweise unter neutralen oder sauren Bedingungen, in Lösung oder als Suspension, in einem polaren Lösungsmittel wie Wasser, Methanol, Ethanol, Eisessig, Salzsäure, Schwefelsäure oder Phosphorsäure bei Temperaturen zwischen - 5 °C und 10 °C durch. Zur Diazotierung kommen vorwiegend anorganische Salze oder organische Ester der salpetrigen Säure in Frage, wie Natrium- oder Kaliumnitrit oder Amylnitrit. Die so erhaltene Lösung des

Diazoniumsalzes tropft man zu einer wässrigen Lösung, die Kupfer(I)-cyanid und ein Cyanidsalz wie Natrium- oder Kaliumcyanid sowie eine Base wie Natrium- oder Kaliumcarbonat oder -hydrogencarbonat enthält. Die Lösung wird während des Zutropfens auf einer Temperatur von 20 bis 100 °C, vorzugsweise 50 bis 100 °C, gehalten.

Nitrile der allgemeinen Formel VII, in der A ein Wasserstoffatom bedeutet (= allgemeine Formel VIIa) lassen sich zu Nitrilen der allgemeinen Formel VII, in der A eine Alkyl-, Alkenyl-, Alkinyl- oder Cycloalkylgruppe bedeutet (= allgemeine Formel VIIb, in der R′ die gleiche Bedeutung wie R mit Ausnahme des Wasserstoffatoms hat) alkylieren.

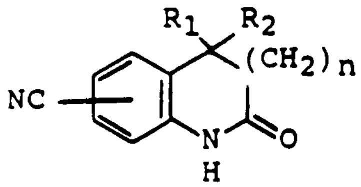

(VIIa)

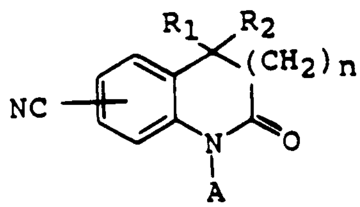

(VIIb)

Diese Alkylierungen werden vorzugsweise in einem Lösungsmittel wie Aceton, Methylethylketon, Ether, Benzol, Toluol oder Dimethylformamid bei Temperaturen zwischen -30 °C und +100 °C, vorzugsweise bei 0-80 °C in Gegenwart einer Base wie Alkylhalogeniden, Alkenylhalogeniden, Alkinylhalogeniden, Cycloalkylhalogeniden oder den entsprechenden -sulfaten durchgeführt. Zur Beschleunigung der Reaktion können katalytische Mengen eines Kronenethers zugegeben werden.

Die Nitrile der allgemeinen Formel VII werden nun zu den Carbonsäuren der allgemeinen Formel IV verseift. Die Hydrolyse wird zweckmäßigerweise entweder in Gegenwart einer Säure wie Salzsäure, Schwefelsäure, Trichloressigsäure oder in Gegenwart einer Base wie Natriumhydroxid oder Kaliumhydroxid in einem geeigneten Lösungsmittel wie Wasser oder in Mischungen wie Wasser/Ethanol oder Wasser/Methanol oder Wasser/Dioxan bei Temperaturen zwischen 0 und 120 °C, bevorzugt beim Siedepunkt des Gemisches, durchgeführt.

Verbindungen der allgemeinen Formel I können auch in andere Verbindungen der allgemeinen Formel I umgewandelt werden. Dies trifft zu für

a) die Alkylierung von Verbindungen der allgemeinen Formel I, in der $R_3$ eine Gruppe der allgemeinen Formel II bedeutet, wobei einer oder mehrere der Substituenten $R_4$, $R_5$ oder $R_6$ eine Hydroxy- oder Mercaptogruppe bedeuten, zu den entsprechenden Alkoxy- oder Alkylthioverbindungen.

Diese Reaktionen werden vorzugsweise in einem Lösungsmittel wie Aceton, Ether, Benzol, Toluol oder Dimethylformamid bei Temperaturen zwischen - 30 °C und + 100 °C, vorzugsweise bei Raumtemperatur in Gegenwart einer Base wie Kaliumhydroxid und eines Alkylierungsmittels wie Alkylhalogeniden oder Alkylsulfaten durchgeführt

b) die Herstellung von Verbindungen der allgemeinen Formel I, in der $R_3$ einen Rest der allgemeinen Formel II bedeutet und $R_4$ eine Alkylsulfinyl- oder Alkylsulfonylgruppe darstellt, durch nachträgliche Oxidation einer Verbindung, in der $R_4$ eine Alkylmercaptogruppe ist. Die Oxidation wird vorzugsweise in einem Lösungsmittel oder Lösungsmittelgemisch, z. B. Wasser, Wasser/Pyridin, Aceton, Eisessig, verdünnter Schwefelsäure oder Trifluoressigsäure, je nach dem verwendeten Oxidationsmittel, zweckmäßigerweise bei Temperaturen zwischen - 80 °C und 100 °C durchgeführt.

Zur Herstellung einer Alkylsulfinylverbindung der allgemeinen Formel I wird die Oxidation zweckmäßigerweise mit einem Äquivalent des verwendeten Oxidationsmittels durchgeführt, z. B. mit Wasserstoffperoxid in Eisessig, Trifluoressigsäure oder Ameisensäure bei 0 bis 20 °C oder in Aceton bei 0 bis 60 °C, mit einer Persäure wie Perameisensäure in Eisessig oder Trifluoressigsäure bei 0 bis 50 °C oder mit m-Chlorperbenzoesäure in Methylenchlorid oder Chloroform bei - 20 °C bis 60 °C, mit Natriummetaperjodat in wässrigem Methanol oder Ethanol bei - 15 °C bis 25 °C, mit Brom in Eisessig oder wässriger Essigsäure, mit N-Bromsuccinimid in Ethanol, mit tert.-Butyl-hypochlorit in Methanol bei - 80

°C bis - 30 °C, mit Jodbenzodichlorid in wässrigem Pyridin bei 0 bis 50 °C, mit Salpetersäure in Eisessig bei 0 bis 20 °C, mit Chromsäure in Eisessig oder in Aceton bei 0 bis 20 °C und mit Sulfurylchlorid in Methylenchlorid bei - 70 °C, der hierbei erhaltene Thioether-Chlorkomplex wird zweckmäßigerweise mit wässrigem Ethanol hydrolysiert.

Zur Herstellung einer Alkylsulfonylverbindung der allgemeinen Formel I wird die Oxidation zweckmäßigerweise mit zwei oder mehr Äquivalenten des verwendeten Oxidationsmittels durchgeführt, z. B. Wasserstoffperoxid in Eisessig, Trifluoressigsäure oder in Ameisensäure bei 20 bis 100 °C oder in Aceton bei 0 bis 60 °C; mit einer Persäure wie Perameisensäure oder m-Chlorperbenzoesäure in Eisessig, Trifluoressigsäure, Methylenchlorid oder Chloroform bei Temperaturen zwischen 0 und 60 °C, mit Salpetersäure in Eisessig bei 0 bis 20 °C, mit Chromsäure oder Kaliumpermanganat in Eisessig, Wasser/Schwefelsäure oder in Aceton bei 0 bis 20 °C.

c) Die Herstellung von Verbindungen der allgemeinen Formel I, in der $R_3$ einen Rest der allgemeinen Formel II bedeutet und $R_4$ eine Alkansulfonyloxy-, Trifluormethansulfonyloxy-, Alkansulfonylamino- oder Trifluormethansulfonylaminogruppe darstellt, durch die nachträgliche Umsetzung einer Verbindung, in der $R_4$ eine Hydroxygruppe ist mit einer Sulfonsäure der allgemeinen Formel IX

$R_8 - SO_3H$ (IX)

in der $R_8$ eine Alkylgruppe oder die Trifluormethylgruppe darstellt, in Gegenwart eines wasserentziehenden und/oder die Säure oder das Amin aktivierenden Mittels oder mit deren reaktionsfähigen Derivaten.

Die Umsetzung wird zweckmäßigerweise in einem Lösungsmittel oder Lösungsmittelgemisch wie Methylenchlorid, Ether, Tetrahydrofuran, Dioxan oder Benzol, gegebenenfalls in Gegenwart eines säurebindenden Mittels wie Natriumcarbonat, Triethylamin oder Pyridin, wobei die beiden letzteren gleichzeitig auch als Lösungsmittel verwendet werden können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der allgemeinen Formel VII, z. B. mit deren Anhydrid oder Halogenid, wie Methansulfonsäurechlorid oder Ethansulfonsäurechlorid, bei Temperaturen zwischen 0 und 100 °C, vorzugsweise bei Temperaturen zwischen Raumtemperatur und 50 °C, durchgeführt.

d) Die Herstellung von Verbindungen der allgemeinen Formel I, in der $R_3$ einen Rest der allgemeinen Formel II bedeutet und $R_4$ eine durch eine Amino-, Alkylamino- oder Dialkylaminogruppe substituierte Carbonylgruppe darstellt, durch die nachträgliche Umsetzung einer Verbindung in der $R_4$ eine Carboxylgruppe darstellt, oder einem reaktionsfähigen Derivat hiervon, wie z. B. Ester oder Säurechlorid mit einem Amin der allgemeinen Formel X

$HNR_9R_{10}$ (X)

in der $R_9$ und $R_{10}$, die gleich oder verschieden sein können, Wasserstoffatome oder Alkylgruppen darstellen, oder mit einem reaktionsfähigen Derivat hiervon, falls $R_4$ die Carboxylgruppe darstellt. Die Umsetzung wird zweckmäßigerweise in einem Lösungsmittel wie Methylenchlorid, Ethanol, Chloroform, Tetrachlorkohlenstoff, Ether, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z. B. in Gegenwart von Chlorameisensäureethylester, Thionylchlorid, Phosphortrichlorid, Phosphorpentoxid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triethylamin oder Pyridin, welche gleichzeitig als Lösungsmittel dienen können, bei Temperaturen zwischen - 25 und 250 °C, vorzugsweise jedoch bei Temperaturen zwischen - 10 °C und der Siedetemperatur des verwendeten Lösungsmittels, durchgeführt, desweiteren kann während der Umsetzung entstehendes Wasser durch azeotrope Destillation, z.B. durch Erhitzen mit Toluol an Wasserabscheider, oder durch Zugabe eines Trockenmittels wie Magnesiumsulfat oder Molekularsieb abgetrennt werden.

Besonders vorteilhaft wird jedoch die Umsetzung mit einem entsprechenden Halogenid, z. B. dem Carbonsäure- oder Sulfonsäurechlorid, und einem entsprechenden Amin, wobei dieses gleichzeitig als Lösungsmittel dienen kann, bei Temperaturen zwischen 0 und 50 °C durchgeführt.

e) Die Alkylierung von Verbindungen der allgemeinen Formel I′

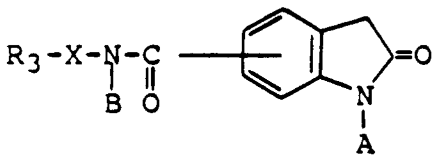

(I')

in der A, B, $R_3$ und X die oben angegebenen Bedeutungen haben, zu Verbindungen der allgemeinen Formel II′

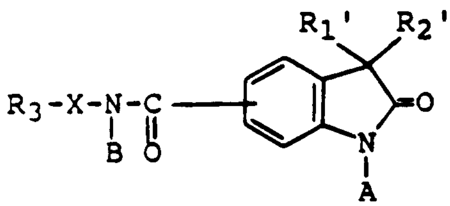

(II')

in der A, B, $R_3$ und X die oben angegebenen Bedeutungen haben und $R_1'$ und $R_2'$ Alkyl-, Alkenyl- oder Cycloalkylgruppen bedeuten, oder zusammen mit dem C-Atom, an das sie gebunden sind, einen spirocyclischen Alkylring bedeuten.

Diese Reaktionen werden vorzugsweise so durchgeführt, daß man die freien NH-Positionen in der allgemeinen Formel I′ schützt, vorzugsweise durch eine Acetylgruppe, und dann in einem Lösungsmittel wie Aceton, Ether, Benzol, Toluol oder Dimethylformamid bei Temperaturen zwischen - 30 °C und + 100 °C, vorzugsweise bei Raumtemperatur in Gegenwart einer Base wie Kaliumhydroxid und eines Alkylierungsmittels der allgemeinen Formel XI

$R_1'$-Hal (XI)

oder der allgemeinen Formel XII

$R_2'$-Hal (XII)

in der $R_1'$ und $R_2'$ die oben angegebenen Bedeutungen haben, alkyliert. Vorzugsweise wird die Alkylierung in Gegenwart eines Phasentransferkatalysators wie N-Benzyltriethylammoniumbromid durchgeführt. Anschließend werden die Acetyl-Schutzgruppen entfernt. Das geschieht bereits bei der Aufarbeitung nach der Alkylierung, die Entacetylierung kann jedoch vervollständigt werden durch kurzzeitiges Erhitzen in einer wässrigen Säure wie Salzsäure oder Schwefelsäure.

f) Die Umsetzung von Verbindungen der allgemeinen Formel I, in der $R_2$ die Alkoxycarbonylgruppe bedeutet, zu Verbindungen der allgemeinen Formel I, in der $R_2$ die Hydrazinocarbonylgruppe bedeutet. Dazu setzt man in einem Lösungsmittel wie Ethanol, Methanol oder Eisessig mit einem geringen Überschuß an Hydrazinhydrat bei Temperaturen zwischen Raumtemperatur und dem Siedepunkt des Lösungsmittels um.

g) Die Oxidation von Verbindungen der allgemeinen Formel I, in der $R_3$ eine Fünf- oder Sechsring mit einem oder mehreren Stickstoffatomen bedeutet, zu den entsprechenden N-Oxiden. Die Oxidation erfolgt vorzugsweise mit einem oder mehreren Äquivalenten eines Oxidationsmittels wie Wasserstoffperoxid in Eisessig, Trifluoressigsäure oder Ameisensäure bei 20 bis 100 °C oder in Aceton bei 0 bis 60 °C, mit einer Persäure wie Perameisensäure oder m-Chlorperbenzoesäure in Eisessig, Trifluoressigsäure oder Methylenchlorid bei Temperaturen zwischen 0 bis 60 °C.

h) Die Alkylierung von Verbindungen der allgemeinen Formel I′

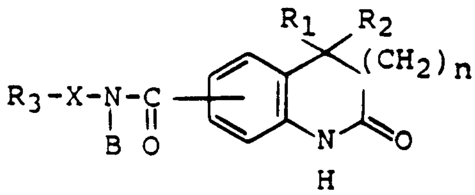

(I')

in der B, $R_1$, $R_2$, $R_3$ und X die oben genannten Bedeutungen haben, mit Alkylierungsmitteln A′-Hal, wobei A′ die gleichen Bedeutungen wie A mit Ausnahme von Wasserstoff hat, zu den Verbindungen der allgemeinen Formel I″,

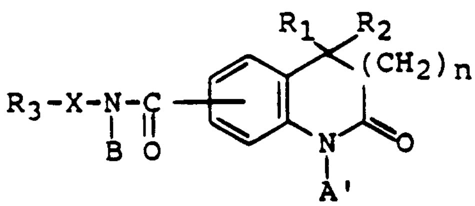

(I")

in der A′, B, $R_1$, $R_2$, $R_3$, X und n die oben genannten Bedeutungen aufweisen. Diese Alkylierungen werden vorzugsweise in einem Lösungsmittel wie Aceton, Methylethylketon, Ether, Benzol, Toluol oder Dimethylformamid bei Temperaturen zwischen -30 °C und 100 °C, vorzugsweise bei 0 °C-80 ° in Gegenwart einer Base wie Natriumhydroxide oder Kaliumcarbonat, und einem geringen Unterschuß des Alkylierungsmittels durchgeführt.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragées ausgeformt oder unter Zugabe entsprechender Hilfsstoffe in Wasser oder Öl, wie z. B. Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citratpuffer, Ethanol, Komplexbildner (wie Ethylendiamintetraessigsäure und deren nicht toxische Salze) und hochmolekulare Polymere (wie flüssiges Polyethylenoxid) zur Viskositätsregulierung. Feste Trägerstoffe sind z. B. Stärke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 10 bis 1500 mg pro Tag bezogen auf 75 kg Körpergewicht appliziert. Bevorzugt ist es, 2- bis 3mal pro Tag 1 bis 2 Tabletten mit einem Wirkstoffgehalt von 5 bis 500 mg zu verabreichen. Die Tabletten können auch retardiert sein, wodurch nur noch einmal pro Tag 1 bis 2 Tabletten mit 20 bis 700 mg Wirkstoff gegeben werden müssen. Der Wirkstoff kann auch durch Injektion 1- bis 8mal pro Tag bzw. durch Dauerinfusion gegeben werden, wobei Mengen von 10 bis 1.000 mg pro Tag normalerweise ausreichen.

**Als Carbonsäuren der allgemeinen Formel IV kommen beispielsweise die folgenden Verbindungen in Frage:**

2,3-Dihydro-3,3-diethyl-2-oxo-(1H)-indol-6-carbonsäure
2,3-Dihydro-3,3-dibutyl-2-oxo-(1H)-indol-6-carbonsäure
2,3-Dihydro-3-methyl-2-oxo-(1H)-indol-6-carbonsäure
2,3-Dihydro-3-ethyl-2-oxo-(1H)-indol-6-carbonsäure

2,3-Dihydro-3-propyl-2-oxo-(1H)-indol-6-carbonsäure
2,3-Dihydro-3-(1-methyl-ethyl)-2-oxo-(1H)-indol-6-carbonsäure
2,3-Dihydro-3-ethoxycarbonyl-3-methyl-2-oxo-(1H)-indol-6-carbonsäure
2,3-Dihydro-3-hydrazinocarbonyl-3-methyl-2-oxo-(1H)-indol-6-carbonsäure
2′,3′-Dihydro-2′-oxo-(1′H)-spiro[1,3′-cyclohexan-indol]-6′-carbonsäure
1,2,3,4-Tetrahydro-4,4-dimethyl-2-oxo-6-chinolincarbonsäure
2′,3′-Dihydro-2′-oxo-(1′H)-spiro[cyclopentan-1,3′-indol]- 5′-carbonsäure

**Als Nitrile der allgemeinen Formel VII kommen beispielsweise die folgenden Verbindungen in Frage:**

2,3-Dihydro-3,3-diethyl-2-oxo-(1H)-indol-6-carbonitril
2,3-Dihydro-3,3-dibutyl-2-oxo-(1H)-indol-6-carbonitril
2,3-Dihydro-3-methyl-2-oxo-(1H)-indol-6-carbonitril
2,3-Dihydro-3-ethyl-2-oxo-(1H)-indol-6-carbonitril
2,3-Dihydro-3-propyl-2-oxo-(1H)-indol-6-carbonitril
2,3-Dihydro-3-(1-methyl-ethyl)-2-oxo-(1H)-indol-6-carbonitril
2,3-Dihydro-3-ethoxycarbonyl-3-methyl-2-oxo-(1H)-indol-6-carbonitril
2,3-Dihydro-3-hydrazinocarbonyl-3-methyl-2-oxo-(1H)-indol-6-carbonitril
2′,3′-Dihydro-2′-oxo-(1′H)-spiro[1,3′-cyclopropan-indol]-6′-carbonitril
2′,3′-Dihydro-2′-oxo-(1′H)-spiro[1,3′-cyclopentan-indol]-6′-carbonitril
2′,3′-Dihydro-2′-oxo-(1′H)-spiro[1,3′-cyclohexan-indol]-6′-carbonitril

**Als Verbindungen der allgemeinen Formel I kommen außer den in den Beispielen genannten Verbindungen die folgenden Verbindungen in Frage:**

1. N-(4-Diethylaminophenyl)-2,3-dihydro-3,3-dimethyl-2-oxo-(1H)-indol-6-carboxamid
2. N-(4-Acetylphenyl)-2,3-dihydro-3,3-dimethyl-2-oxo-(1H)-indol-6-carboxamid
3. N-(4-Ethylphenyl)-2,3-dihydro-3,3-dimethyl-2-oxo-(1H)-indol-6-carboxamid
4. N-(4-Isopropylphenyl)-2,3-dihydro-3,3-dimethyl-2-oxo-(1H)-indol-6-carboxamid
5. N-(4-(1-Methyl-propyl)phenyl)-2,3-dihydro-3,3-dimethyl-2-oxo-(1H)-indol-6-carboxamid
6. N-(4-Bromphenyl)-2,3-dihydro-3,3-dimethyl-2-oxo-(1H)-indol-6-carboxamid
7. N-(3-Hydroxyphenyl)-2,3-dihydro-3,3-dimethyl-2-oxo-(1H)-indol-6-carboxamid
8. N-(3-Ethoxyphenyl)-2,3-dihydro-3,3-dimethyl-2-oxo-(1H)-indol-6-carboxamid
9. N-(3-Acetylphenyl)-2,3-dihydro-3,3-dimethyl-2-oxo-(1H)-indol-6-carboxamid
10. N-(3-Methylphenyl)-2,3-dihydro-3,3-dimethyl-2-oxo-(1H)-indol-6-carboxamid
11. N-(3-Ethylphenyl)-2,3-dihydro-3,3-dimethyl-2-oxo-(1H)-indol-6-carboxamid
12. N-(3-Hydroxymethylphenyl)-2,3-dihydro-3,3-dimethyl-2-oxo-(1H)-indol-6-carboxamid
13. N-(3-Fluorphenyl)-2,3-dihydro-3,3-dimethyl-2-oxo-(1H)-indol-6-carboxamid
14. N-(3-Bromphenyl)-2,3-dihydro-3,3-dimethyl-2-oxo-(1H)-indol-6-carboxamid
15. N-(2-Aminophenyl)-2,3-dihydro-3,3-dimethyl-2-oxo-(1H)-indol-6-carboxamid
16. N-(2-Acetamidophenyl)-2,3-dihydro-3,3-dimethyl-2-oxo-(1H)-indol-6-carboxamid
17. N-(2-Hydroxyphenyl)-2,3-dihydro-3,3-dimethyl-2-oxo-(1H)-indol-6-carboxamid
18. N-(2-Ethoxyphenyl)-2,3-dihydro-3,3-dimethyl-2-oxo-(1H)-indol-6-carboxamid
19. N-(2-Acetylphenyl)-2,3-dihydro-3,3-dimethyl-2-oxo-(1H)-indol-6-carboxamid
20. N-(2-Methoxycarbonylphenyl)-2,3-dihydro-3,3-dimethyl-2-oxo-(1H)-indol-6-carboxamid
21. N-(2-Ethoxycarbonylphenyl)-2,3-dihydro-3,3-dimethyl-2-oxo-(1H)-indol-6-carboxamid
22. N-(2-Trifluormethylphenyl)-2,3-dihydro-3,3-dimethyl-2-oxo-(1H)-indol-6-carboxamid
23. N-(2-Ethylphenyl)-2,3-dihydro-3,3-dimethyl-2-oxo-(1H)-indol-6-carboxamid
24. N-(2-Hydroxymethylphenyl)-2,3-dihydro-3,3-dimethyl-2-oxo-(1H)-indol-6-carboxamid
25. N-(2-(1-Methyl-propyl)phenyl)-2,3-dihydro-3,3-dimethyl-2-oxo-(1H)-indol-6-carboxamid
26. N-(2-Fluorphenyl)-2,3-dihydro-3,3-dimethyl-2-oxo-(1H)-indol-6-carboxamid
27. N-(2-Bromphenyl)-2,3-dihydro-3,3-dimethyl-2-oxo-(1H)-indol-6-carboxamid
28. N-(2-Cyanophenyl)-2,3-dihydro-3,3-dimethyl-2-oxo-(1H)-indol-6-carboxamid
29. N-(4-(3-Pyridinyloxy)phenyl)-2,3-dihydro-3,3-dimethyl-2-oxo-(1H)-indol-6-carboxamid
30. N-(2-Amino-4-chlorphenyl)-2,3-dihydro-3,3-dimethyl-2-oxo-(1H)-indol-6-carboxamid
31. N-(2-Amino-4-methoxyphenyl)-2,3-dihydro-3,3-dimethyl-2-oxo-(1H)-indol-6-carboxamid
32. N-(2-Amino-6-methylphenyl)-2,3-dihydro-3,3-dimethyl-2-oxo-(1H)-indol-6-carboxamid

33. N-(2-Amino-5-chlorphenyl)-2,3-dihydro-3,3-dimethyl-2-oxo-(1H)-indol-6-carboxamid
34. N-(2-Methyl-5-aminophenyl)-2,3-dihydro-3,3-dimethyl-2-oxo-(1H)-indol-6-carboxamid
35. N-(2-Methyl-3-aminophenyl)-2,3-dihydro-3,3-dimethyl-2-oxo-(1H)-indol-6-carboxamid
36. N-(2-Methoxy-5-aminophenyl)-2,3-dihydro-3,3-dimethyl-2-oxo-(1H)-indol-6-carboxamid
37. N-(2-Amino-4-fluorphenyl)-2,3-dihydro-3,3-dimethyl-2-oxo-(1H)-indol-6-carboxamid
38. N-(2-Methyl-4-aminophenyl)-2,3-dihydro-3,3-dimethyl-2-oxo-(1H)-indol-6-carboxamid
39. N-(2-Methoxy-4-aminophenyl)-2,3-dihydro-3,3-dimethyl-2-oxo-(1H)-indol-6-carboxamid
40. N-(2-Chlor-4-aminophenyl)-2,3-dihydro-3,3-dimethyl-2-oxo-(1H)-indol-6-carboxamid
41. N-(2-Brom-4-aminophenyl)-2,3-dihydro-3,3-dimethyl-2-oxo-(1H)-indol-6-carboxamid
42. N-(2,4-Diaminophenyl)-2,3-dihydro-3,3-dimethyl-2-oxo-(1H)-indol-6-carboxamid
43. N-(2,6-Dichlor-4-aminophenyl)-2,3-dihydro-3,3-dimethyl-2-oxo-(1H)-indol-6-carboxamid
44. N-(2-Hydroxy-5-chlorphenyl)-2,3-dihydro-3,3-dimethyl-2-oxo-(1H)-indol-6-carboxamid
45. N-(2-Hydroxy-5-methylphenyl)-2,3-dihydro-3,3-dimethyl-2-oxo-(1H)-indol-6-carboxamid
46. N-(2-Thenyl)-2,3-dihydro-3,3-dimethyl-2-oxo-(1H)-indol-6-carboxamid
47. N-(2-Thiazolyl)-2,3-dihydro-3,3-dimethyl-2-oxo-(1H)-indol-6-carboxamid
48. N-(5-Methyl-3-isoxazolyl)-2,3-dihydro-3,3-dimethyl-2-oxo-(1H)-indol-6-carboxamid
49. N-(1,3,4-Thiadiazol-2-yl)-2,3-dihydro-3,3-dimethyl-2-oxo-(1H)-indol-6-carboxamid
50. N-(5-Methyl-1,3,4-thiadiazol-2-yl)-2,3-dihydro-3,3-dimethyl-2-oxo-(1H)-indol-6-carboxamid
51. N-(N-Oxy-4-pyridinyl)-2,3-dihydro-3,3-dimethyl-2-oxo-(1H)-indol-6-carboxamid
52. N-(2-Pyrazinyl)-2,3-dihydro-3,3-dimethyl-2-oxo-(1H)-indol-6-carboxamid
53. N-(2-Pyrimidinyl)-2,3-dihydro-3,3-dimethyl-2-oxo-(1H)-indol-6-carboxamid
54. N-(1,2,4-Triazin-3-yl)-2,3-dihydro-3,3-dimethyl-2-oxo-(1H)-indol-6-carboxamid
55. N-(6-Methoxy-3-pyridinyl)-2,3-dihydro-3,3-dimethyl-2-oxo-(1H)-indol-6-carboxamidl
56. N-(6-Propyloxy-3-pyridinyl)-2,3-dihydro-3,3-dimethyl-2-oxo-(1H)-indol-6-carboxamid
57. N-(6-Isopropyloxy-3-pyridinyl)-2,3-dihydro-3,3-dimethyl-2-oxo-(1H)-indol-6-carboxamid
58. N-(6-Allyloxy-3-pyridinyl)-2,3-dihydro-3,3-dimethyl-2-oxo-(1H)-indol-6-carboxamid
59. N-(6-(4-Pyridinyloxy)-3-pyridinyl)-2,3-dihydro-3,3-dimethyl-2-oxo-(1H)-indol-6-carboxamid
60. N-(6-Phenyloxy-3-pyridinyl)-2,3-dihydro-3,3-dimethyl-2-oxo-(1H)-indol-6-carboxamid
61. N-(6-(4-Methoxyphenyloxy-3-pyridinyl)-2,3-dihydro-3,3-dimethyl-2-oxo-(1H)-indol-6-carboxamid
62. N-(6-(3-Trifluormethylphenyloxy)-3-pyridinyl)-2,3-dihydro-3,3-dimethyl-2-oxo-(1H)-indol-6-carboxamid
63. N-(6-(4-Cyanophenyloxy)-3-pyridinyl)-2,3-dihydro-3,3-dimethyl-2-oxo-(1H)-indol-6-carboxamid
64. N-(6-(2-Methoxyphenoxy)-3-pyridinyl)-2,3-dihydro-3,3-dimethyl-2-oxo-(1H)-indol-6-carboxamid
65. N-(6-(3-Methoxyphenoxy)-3-pyridinyl)-2,3-dihydro-3,3-dimethyl-2-oxo-(1H)-indol-6-carboxamid
66. N-(6-(4-methylphenoxy)-3-pyridinyl)-2,3-dihydro-3,3-dimethyl-2-oxo-(1H)-indol-6-carboxamid
67. N-(6-(4-Ethoxycarbonylphenyloxy)-3-pyridinyl)-2,3-dihydro-3,3-dimethyl-2-oxo-(1H)-indol-6-carboxamid
68. N-(2-Benzimidazolyl)-2,3-dihydro-3,3-dimethyl-2-oxo-(1H)-indol-6-carboxamid
69. N-(2-Benzthiazolyl)-2,3-dihydro-3,3-dimethyl-2-oxo-(1H)-indol-6-carboxamid
70. N-(2-Methyl-4-chinolinyl)-2,3-dihydro-3,3-dimethyl-2-oxo-(1H)-indol-6-carboxamid
71. N-(5-Methyl-7-hydroxy-[1,8]-naphthyridin-2-yl)-2,3-dihydro-3,3-dimethyl-2-oxo-(1H)-indol-6-carboxamid
72. N-(1,4-Dihydroxy-5-phthalazinyl)-2,3-dihydro-3,3-dimethyl-2-oxo-(1H)-indol-6-carboxamid
73. N-Phenyl-2′,3′-dihydro-2′-oxo-(1′H)-spiro[1,3-′cyclopropan-indol]-6′-carboxamid
74. N-Phenyl-2′,3′-dihydro-2′-oxo-(1′H)-spiro[1,3′-cyclohexan-indol]-6′-carboxamid
75. 2,3-Dihydro-3,3-dimethyl-N-benzyl-N-phenyl-2-oxo-(1H)-indol-6-carboxamid
76. 2,3-Dihydro-3,3-dimethyl-1-ethyl-N-ethyl-N-(4-fluorphenyl)-2-oxo-(1H)-indol-6-carboxamid
77. N-(4-Biphenyl)-2,3-dihydro-3,3-dimethyl-2-oxo-(1H)-indol-6-carboxamid, Fp. 288-291 °C.
78. N-(4-Methylsulfonylamino-phenyl)-2,3-dihydro-3,3-dimethyl-2-oxo-(1H)-indol-6-carboxamid
79. N-(4-Phenylsulfonylamino-phenyl)-2,3-dihydro-3,3-dimethyl-2-oxo-(1H)-indol-6-carboxamid

Beispiel 1

**2,3-Dihydro-3,3-dimethyl-N-phenyl-2-oxo-(1H)-indol-6-carboxamid**

a) 25 g (0,142 Mol) 6-Amino-1,3-dihydro-3,3-dimethyl-(2H)-indolin-2-on in 250 ml 2N HCl wurden unter Eiskühlung innerhalb von 15 Minuten mit einer Lösung aus 10,3 g (0,15 Mol) $NaNO_2$ in 20 ml Wasser versetzt und nach der Zugabe 15 Minuten gerührt. Nach Versetzen mit 2,13 g (0,036 Mol) Harnstoff wurde die klare Lösung nach weiteren 10 Minuten Rühren zu einer auf 50 °C erwärmten Lösung aus

24,4 g (0,5 Mol) NaCN, 15,2 g (0,17 Mol) CuCN und 22,6 g (0,21 Mol) $Na_2CO_3$ in 820 ml Wasser getropft und 5 Minuten auf 90 °C erhitzt.

Nach dem Abkühlen wurde der Niederschlag abgesaugt, mit Wasser gewaschen und nach Trocknung aus Ethanol umkristallisiert. Man erhielt 15 g (57 %) 2,3-Dihydro-3,3-dimethyl-2-oxo(1H)-indol-6-carbonitril als farblose Kristalle mit dem Fp. 243 bis 246 °C.

b) 12,2 g 2,3-Dihydro-3,3-dimethyl-2-oxo-1H-indol-6-carbonitril wurden in 150 ml 20 % KOH 3 Stunden unter Rückfluß zum Sieden erhitzt, abgekühlt, mit conc. Salzsäure neutralisiert, das Kristallisat abgesaugt und mit Wasser gewaschen. Man erhielt 13,1 g (98 %) 2,3-Dihydro-3,3-dimethyl-2-oxo-(1H)-indol-6-carbonsäure mit dem Fp. 295 bis 300 °C (Zers.).

c) 8 g 2,3-Dihydro-3,3-dimethyl-2-ozo-(1H)-indol-6-carbonsäure und 1 Tropfen Dimethylformamid erhitzte man in 50 ml Thionylchlorid 1 Stunde unter Rückfluß zum Sieden, wobei die Substanz in Lösung geht. Man entfernte das Thionylchlorid i. Vak., suspendierte den Rückstand in Dichlormethan und tropfte ihn zu einer Lösung von 1,6 ml Anilin und 2,7 ml Triethylamin in 30 ml Dichlormethan bei 15 °C Innentemperatur. Man rührte noch 15 Minuten bei Raumtemperatur, entfernte das Lösungsmittel i. Vak., digerierte der Rückstand mit Wasser, saugte ab und kristallisierte aus Essigester um. Man erhielt 2,8 g (56 %) der Titelverbindung mit dem Fp. 205 bis 206 °C.

Analog dem Beispiel 1 erhielt man durch Umsetzung mit den angegebenen Aminen anstelle des Anilins die folgenden Verbindungen:

| | Bezeichnung | Ausbeute Fp. | umkrist. aus |
|---|---|---|---|
| 2 | 2,3-Dihydro-3,3-dimethyl-N-(3-trifluormethyl-phenyl)-2-oxo-indol-6-carboxamid aus 3-Tri-fluormethylanilin | 34 % 182-186°C | Essig-ester |
| 3 | 2,3-Dihydro-3,3-dimethyl-N-(4-pyridinyl)-2-oxo-(1H)-indol-6-carboxamid aus 4-Pyridinamin | 53 % 336-341°C | Methanol |
| 4 | 2,3-Dihydro-3,3-dimethyl-N-[6-(3-pyridinyloxy)-3-pyridinyl]-2-oxo-(1H)-indol-6-carboxamid aus 2-(3-Pyridinyloxy)-5-pyridinamin | 48 % 198-200°C | Essig-ester |
| 5 | 2,3-Dihydro-3,3-dimethyl-N-[4-(4,5-dihydro-4-methyl-6-oxo-(1H)-3-pyridazinyl)-phenyl]-2-oxo-(1H)-indol-6-carboxamid aus 4-(4,5-Dihydro-4-methyl-6-oxo-(1H)-3-pyridazinyl)-anilin | 78 % 273-278°C | Isopropanol |
| 6 | 2,3-Dihydro-3,3-dimethyl-N-(4-nitrophenyl)-2-oxo-(1H)-indol-6-carboxamid aus 4-Nitroanilin | 62 % 268-271°C | Ethanol |
| 7 | 2,3-Dihydro-3,3-dimethyl-N-(4-dimethylaminophenyl)-2-oxo-(1H)-indol-6-carbox-amid x 0.5 $H_2O$ aus 4-Dimethylaminoanilin | 60 % 333-335°C | DMF/Wasser |

| | Bezeichnung | Ausbeute Fp. | umkrist. aus |
|---|---|---|---|
| 8 | 2,3-Dihydro-3,3-dimethyl-N-(4-hydroxy-2-methyl-phenyl)-2-oxo(1H)-indol-6-carboxamid x $H_2O$ aus 4-Hydroxy-2-methylanilin | 17 % 110-130°C | Essigester |
| 9 | 2,3-Dihydro-3,3-dimethyl-N-(2-(2-pyridinyl)ethyl)-2-oxo-(1H)-indol-6-carboxamid aus 2-(2-Pyridinyl)ethanamin | 70 % 185-187°C | Essigester |
| 10 | 2,3-Dihydro-3,3-dimethyl-N-(3-(1,2,4-triazolo[4,3-a]-pyridinyl))-2-oxo-(1H)-indol-6-carboxamid x0.5 $H_2O$ aus 1,2,4-Triazolo[4,3-a]-pyridin-3-amin | 42 % 310-312°C | Methanol |
| 11 | 2,3-Dihydro-3,3-dimethyl-N-(4-methylphenyl)-2-oxo-(1H)-indol-6-carboxamid aus 4-Methylanilin | 48 % 245-247°C | Ethanol |
| 12 | 2,3-Dihydro-3,3-dimethyl-N-(4-fluorphenyl)-2-oxo-(1H)-indol-6-carboxamid aus 4-Fluoranilin | 64 % 235-236°C | Ethanol |
| 13 | 2,3-Dihydro-3,3-dimethyl-N-(4-cyanophenyl)-2-oxo-(1H)-indol-6-carboxamid aus 4-Cyanoanilin | 44 % 264-266°C | Essigester |
| 14 | 2,3-Dihydro-3,3-dimethyl-N-(3-methoxyphenyl)-2-oxo-(1H)-indol-6-carboxamid aus Methyoxyanilin | 68 % 211-213°C | Essigester |
| 15 | 2,3-Dihydro-3,3-dimethyl-N-(3-chlorphenyl)-2-oxo-(1H)-indol-6-carboxamid aus 3-Chloranilin | 46 % 233-235°C | Essigester |
| 16 | 2,3-Dihydro-3,3-dimethyl-N-(2-methylphenyl)-2-oxo-(1H)-indol-6-carboxamid aus 2-Methylanilin | 45 % 219-221°C | Essigester |
| 17 | 2,3-Dihydro-3,3-dimethyl-N-(4-hydroxyphenyl)-2-oxo-(1H)-indol-6-carboxamid aus 4-Hydroxyanilin | 58 % 218-221°C | Essigester |

| | Bezeichnung | Ausbeute Fp. | umkrist. aus |
|---|---|---|---|
| 18 | 2,3-Dihydro-3,3-dimethyl-N-(4-methoxyphenyl)-2-oxo-(1H)-indol-6-carboxamid aus 4-Methoxyanilin | 62 % 241-242°C | Ethanol/Wasser |
| 19 | 2,3-Dihydro-3,3-dimethyl-N-(4-ethoxyphenyl)-2-oxo-(1H)-indol-6-carboxamid aus 4-Ethoxyanilin | | |
| 20 | 2,3-Dihydro-3,3-dimethyl-N-(4-allyloxyphenyl)-2-oxo-(1H)-indol-6-carboxamid aus Allyloxyanilin | | |
| 21 | 2,3-Dihydro-3,3-dimethyl-N-(3,4-methylendioxy-phenyl)-2-oxo-(1H)-indol-6-carboxamid aus 3,4-Methylendioxyanilin | 34 % 266-271°C | Ethanol |
| 22 | 2,3-Dihydro-3,3-dimethyl-N-(3,4-ethylendioxyphenyl)-2-oxo-(1H)-indol-6-carboxamid aus 3,4-Ethylendioxyanilin | 25 % 275-278°C | Ethanol |
| 23 | 2,3-Dihydro-3,3-dimethyl-N-(4-(5,6,7,8-tetrahydro-naphthyl))-2-oxo-(1H)-indol-6-carboxamid aus 2-(5,6,7,8-Tetrahydro-naphthyl)amin | | |
| 24 | 2,3-Dihydro-3,3-dimethyl-N-(5-(1H)-tetrazolyl)-2-oxo-(1H)-indol-6-carboxamid aus 5-(1H)-Tetrazolylamin | | |
| 25 | 2,3-Dihydro-3,3-dimethyl-N-(3-(1H)-(1,2,4-triazolyl))-2-oxo-(1H)-indol-6-carboxamid aus 3-(1H)-(1,2,4-Triazol-yl)amin | | |
| 26 | 2,3-Dihydro-3,3-dimethyl-N-(2-hydroxy-4-methyl-phenyl)-2-oxo-(1H)-indol-6-carboxamid aus 2-Hydroxy-4-methylanilin | | |

| | Bezeichnung | Ausbeute Fp. | umkrist. aus |
|---|---|---|---|
| 27 | 2,3-Dihydro-3,3-dimethyl-N-(3-cyanophenyl)-2-oxo-(1H)-indol-6-carboxamid aus 3-Cyanoanilin | 67 % 304-307°C | Ethanol/ Eisessig |
| 28 | 2,3-Dihydro-3,3-dimethyl-N-(4-ethoxycarbonylmethyloxyphenyl)-2-oxo-(1H)-indol-6-carboxamid aus 4-(Ethoxycarbonyl-methyloxy)anilin | 44 % 204-206°C | Essigester |

Beispiel 29

2,3-Dihydro-3,3-dimethyl-N-(4-aminophenyl)-2-oxo-(1H)-indol-6-carboxamid

2.6 g (8 mmol) 2,3-Dihydro-3,3-dimethyl-N-(4-nitrophenyl)-2-oxo-(1H)-indol-6-carboxamid (aus Beispiel 6) in 50 ml Methanol hydrierte man in Gegenwart von 0.3 g 10 % Pd auf Kohle bei Normaldruck und Raumtemperatur. Nach 2 h saugte man ab, entfernte das Lösungsmittel i.Vak. und reinigte den Rückstand säulenchromatographisch (Kieselgel, Laufmittel Dichlormethan/mit Ammoniak ges. Methanol = 95:5). Man entfernte das Lösungsmittel im Vak., digerierte den Rückstand mit Essigester, saugte ab und trocknete den Rückstand bei 100 °C i. Vakuum. Man erhielt 1.40 g (61 %) der Titelverbindung mit dem Fp. 216-218 °C, der pro Mol noch 0.5 Mol Wasser anhaftete.

Beispiel 30

2,3-Dihydro-3,3-dimethyl-N-(4-methoxyphenyl)-2-oxo-(1H)-indol-5-carboxamid

2.3 g (10 mmol) 2,3-Dihydro-3,3-dimethyl-2-oxo-(1H)-indol-5-carbonsäure, 1 Tropfen DMF und 15 ml Thionylchlorid erhitzte man 30 min unter Rückfluß zum Sieden. Man entfernte das Thionylchlorid i. Vakuum. Das Rohprodukte wurde ohne weitere Reinigung eingesetzt. Man tropfte das in 20 ml Dichlormethan gelöste Rohprodukt zu einer Lösung von 2.5 g (20 mmol) p-Anisidin in 50 ml Dichlormethan unter Eiskühlung. Nach 15 min gab man 50 ml Wasser zu, trennte die organische Phase ab und entfernte das Lösungsmittel i. Vakuum. Den Rückstand reinigte man säulenchromatographisch (400 ml Kieselgel 60, Dichlormethan/Methanol = 20:1). Reine Fraktionen dampfte man i.Vak. ein, digerierte den Rückstand mit Ether, saugte ab und trocknete i. Vak. bei 100 °C. Man erhielt 1.9 g (61 %) der Titelverbindung mit dem Fp. 169-171 °C.

Analog dem Beispiel 30 stellte man durch Umsetzung der 2,3-Dihydro-3,3-dimethyl-2-oxo(1H)-indol-5-carbonsäure mit den angegebenen Anilinen die folgenden Beispielverbindungen her:

| | Bezeichnung | Ausbeute Fp. | umkrist. aus |
|---|---|---|---|
| 31 | 2,3-Dihydro-3,3-dimethyl-N-(4-cyanophenyl)-2-oxo-(1H)-indol-5-carboxamid aus 4-Cyanoanilin | | |
| 32 | 2,3-Dihydro-3,3-dimethyl-N-(3-trifluormethyl-phenyl)-2-oxo-(1H)-indol-5-carboxamid aus 4-Trifluormethylanilin | | |
| 33 | 2,3-Dihydro-3,3-dimethyl-N-(4-ethoxycarbonylmethyloxyphenyl)-2-oxo-(1H)-indol-5-carboxamid aus 4-(Ethoxycarbonylmethyloxy) anilin | 50 % 127-129 °C | Essigester |

Beispiel 34

2,3-Dihydro-3,3-dimethyl-1-ethyl-N-phenyl-2-oxo-(1H)-indol-6-carboxamid

a) 3.1 ml (0.038 mol) Ethyliodid tropfte man bei Raumtemperatur zu 5.9 g (0.032 mol) 2,3-Dihydro-3,3-dimethyl-2-oxo(1H)-indol-6-carbonitril (aus Beispiel 1a) und 5.3 g (0.038 mol) $K_2CO_3$ in 50 ml Dimethylformamid. Man rührte noch 2 h bei 40 °C, goß in 220 ml Wasser und saugte den Niederschlag ab: 6.7 g (100 %) 2,3-Diyhdro-3,3-dimethyl-1-ethyl-2-oxo-(1H)-indol-6-carbonitrilmit dem Fp. 81-83 °C.

b) Nach der Vorschrift von Beispiel 1b erhielt man daraus 7.3 g (98 %) 2,3-Dihydro-3,3-dimethyl-1-ethyl-2-oxo-(1H)-indol-6-carbonsäure mit dem Fp. 228-231 °C.

c) Nach der Vorschrift von Beispiel 1c erhielt man aus 3.8 g (0.015 mol) dieser Substanz 3.6 g (78 %) der Titelverbindung mit dem Fp. 227-230 °C.

Beispiel 35

2,3-Dihydro-3,3-dimethyl-1-ethyl-N-(4-fluorphenyl)-2-oxo-(1H)-indol-6-carboxamid

Durch Umsetzung der in Beispiel 34 b hergestellten 2,3-Dihydro-3,3-dimethyl-1-ethyl-2-oxo-(1H)-indol-6-carbonsäure mit 4-Fluoranilin analog dem Beispiel 1 c erhielt man 58 % der Titelverbindung mit dem Fp. 179-181 °C nach Umkristallisation aus Essigester.

Beispiel 36

2,3-Dihydro-3,3-dimethyl-1-ethyl-N-(4-cyanophenyl)-2-oxo-(1H)-indol-6-carboxamid

Analog dem Beispiel 35 erhielt man durch Umsetzung mit 4-Cyanoanilin 63 % der Titelverbindung mit dem Fp. 211-213 °C nach Umkristallisation aus Essigester.

Beispiel 37

2,3-Dihydro-1,3,3-trimethyl-N-phenyl-2-oxo-(1H)-indol-6-carboxamid

2.8 g 2,3-Dihydro-3,3-dimethyl-N-phenyl-2-oxo-(1H)-indol-6-carboxamid (10 mmol), 2.8 g (20 mmol) Methyliodid und 2.5 g $K_2CO_3$ (20 mmol) rührte man in 50 ml Dimethylformamid 3 h auf 70 °C. Nach dem Abkühlen versetzte man mit Wasser, dekantierte und versetzte wieder mit Wasser. Den Rückstand reinigte man säulenchromatographisch (RP-18; Methanol:Wasser:$NH_4OH$ = 80:20:1). Reine Fraktionen wurden bis zur beginnenden Kristallisation eingedampft, anschließend abgesaugt und mit Wasser gewaschen. Man erhielt 2.3 g der Titelverbindung (78 %) mit dem Fp. 184-186 °C.

Beispiel 38

2,3-Dihydro-3,3-dimethyl-1-propyl-N-phenyl-2-oxo-(1H)-indol-6-carboxamid erhielt man in 65 % Ausbeute analog dem Beispiel 37 durch Umsetzung mit Propyliodid. Fp. 160-162 °C.

Beispiel 39

2,3-Dihydro-3,3-dimethyl-1-allyl-N-phenyl-2-oxo-(1H)-indol-6-carboxamid erhielt man in 63 % Ausbeute analog dem Beispiel 37 durch Umsetzung mit Allylbromid. Fp. 186-188 °C.

Beispiel 40

2,3-Dihydro-3,3-dimethyl-1-(2-methylpropyl)-N-phenyl-2-oxo-(1H)-indol-6-carboxamid erhielt man in 53 % Ausbeute analog dem Beispiel 37 durch Umsetzung mit Isobutyliodid, Fp. 143-144 °C.

Beispiel 41

2,3-Dihydro-3,3-dimethyl-N-ethyl-N-phenyl-2-oxo-(1H)-indol-6-carboxamid erhielt man in 48 % Ausbeute analog dem Beispiel 1c durch Umsetzung mit N-Ethylanilin, Fp. 221-223 °C.

Beispiel 42

4,4-Dimethyl-1,2,3,4-tetrahydro-N-phenyl-2-oxo-7-chinolin-carboxamid

a) Nach der Vorschrift aus Beispiel 1 a erhielt man aus 3,4-Dihydro-(1H)-chinolin-2-on 4.0 g (63 %) 4,4-Dimethyl-1,2,3,4-tetrahydro-2-oxo-7-chinolin-carbonitril mit dem Fp. 207-209 °C.
b) Nach der Vorschrift aus Beispiel 1 b erhielt man daraus quantitativ 4,4-Dimethyl-1,2,3,4-tetrahydro-2-oxo-7-chinolincarbonsäure mit dem Fp. 314-316 °C.
c) 2.7 g (12.3 mmol) 4,4-Dimethyl-1,2,3,4-tetrahydro-2-oxo-7-chinolincarbonsäure gab man zu einer Lösung von 3.8 g (18.5 mmol) N,N'-Dicyclohexylcarbodiimid und 1.1 ml (12.3 mmol) Anilin in 80 ml Dichlormethan. Man rührte 4 h bei Raumtemperatur, saugte den Niederschlag ab und kristallisierte aus Ethanol um. Man erhielt 1.2 g der Titelverbindung mit dem Fp. 249-251 °C.

Analog dem Beispiel 42 stellte man durch Umsetzung der 4,4-Dimethyl-1,2,3,4-tetrahydro-2-oxo-7-chinolincarbonsäure und den angegebenen Anilinen die folgenden Beispielverbindung her:

| | Bezeichnung | Ausbeute Fp. | umkrist. aus |
|---|---|---|---|
| 43 | 4,4-Dimethyl-1,2,3,4-tetrahydro-N-(3-trifluormethylphenyl)-2-oxo-7-chinolincarboxamid aus 3-Trifluormethylanilin | 18 % 275-277 °C | Ethanol |
| 44 | 4,4-Dimethyl-1,2,3,4-tetrahydro-N-(4-cyanophenyl)-2-oxo-7-chinolin-carboxamid aus 4-Cyanoanilin | 10 % 298-301 °C | Ethanol |
| 45 | 4,4-Dimethyl-1,2,3,4-tetrahydro-N-(4-methoxyphenyl)-2-oxo-7-chinolincarboxamid aus 4-Methoxyanilin | 43 % 257-259 °C | Ethanol |
| 46 | 4,4-Dimethyl-1,2,3,4-tetrahydro-N-(4-pyridinyl)-2-oxo-7-chinolincarboxamid aus 4-Pyridinamin | 20 % 306-309 °C | Ethanol |
| 47 | 4,4-Dimethyl-1,2,3,4-tetrahydro-N-ethyl-N-phenyl-2-oxo-7-chinolincarboxamid aus N-Ethylanilin | 11 % 239-241 °C | Ethanol |

Beispiel 48

2', 3'-Dihydro-N-(3-methoxyphenyl)-2'-oxo-spiro[cyclopentan-1,3'-(1'H)-indol]-6'-carboxamid

a) Analog dem Beispiel 1a erhielt man aus 6'-Amino-1',3'-dihydro-spiro[cyclopentan-1,3'-(2H)-indol]-2'-on in 30 % Ausbeute 2',3'-Dihydro-2'-oxo-spiro[cyclopentan-1,3'-(1'H)-indol]-6'-carbonitril mit dem Fp. 178-182 °C als orangefarbene Kristalle.
b) Daraus erhielt man analog dem Beispiel 1b in 88 % Ausbeute 2',3'-Dihydro-2'-oxo-spiro[cyclopentan-1,3'-(1'H)-indol]-6'-carbonsäure mit dem Fp. 272-276 °C als orangefarbene Kristalle.
c) Analog dem Beispiel 1c erhielt man daraus mit 3-Methoxyanilin die Titelverbindung in 34 % Ausbeute mit dem Fp. 227 °C.

Beispiel 49

4,4-Dimethyl-1,2,3,4-tetrahydro-N-(4-fluorphenyl)-2-oxo-7-chinolincarboxamid

mit dem Fp. 281-284 °C erhielt man in 30 % Ausbeute analog dem Beispiel 42 durch Umsetzung mit 4-Fluoranilin.

Beispiel 50

1-Benzyl-2,3-Dihydro-3,3-dimethyl-N-phenyl-2-oxo-(1H)-indol-6-carboxamid

mit dem Fp. 84-88 °C erhielt man in 58 % Ausbeute analog dem Beispiel 37 durch Umsetzung mit Benzylbromid.

Analog dem Beispiel 1 erhielt man durch Umsetzung mit den angegebenen Aminen anstellte des Anilins die folgenden Verbindungen:

| | Bezeichnung | Ausb. Fp. | Lsg.mittel |
|---|---|---|---|
| 51 | 2,3-Dihydro-3,3-dimethyl-N-(2-pyridinyl)-2-oxo-(1H)-indol-6-carboxamid aus 2-Pyridinamin | 18 % 267-269°C | Ethanol |
| 52 | 2,3-Dihydro-3,3-dimethyl-N-(3-pyridinyl)-2-oxo-(1H)-indol-6-carboxamid aus 3-Pyridinamin | 45 % 227-229°C | Ethanol |
| 53 | 2,3-Dihydro-3,3-dimethyl-N-(2-chlorphenyl)-2-oxo-(1H)-indol-6-carboxamid aus 2-Chloranilin | 48 % 235-236°C | Ethanol |
| 54 | 2,3-Dihydro-3,3-dimethyl-N-(2-methoxyphenyl)-2-oxo-(1H)-indol-6-carboxamid aus 2-Methoxyanilin | 30 % 221-223°C | Essigester |
| 55 | 2,3-Dihydro-3,3-dimethyl-N-(4-(3-ethoxycarbonyl-propyloxy)-phenyl)-2-oxo-(1H)-indol-6-carboxamid aus 4-(3-Ethoxycarbonyl-propyloxy)-anilin | 33 % 165-166°C | Ethanol |
| 56 | 2,3-Dihydro-3,3-dimethyl-N-(4-chlorphenyl)-2-oxo-(1H)-indol-6-carboxamid aus 4-Chloranilin | 66 % 249-250°C | Ethanol/ Wasser |
| 57 | 2,3-Dihydro-3,3-dimethyl-N-(3-nitrophenyl)-2-oxo-(1H)-indol-6-carboxamid aus 3-Nitroanilin | 68 % 301-303°C | Ethanol/ Eisessig |
| 58 | 2,3-Dihydro-3,3-dimethyl-N-(2,6-dichlorphenyl)-2-oxo-(1H)-indol-6-carboxamid aus 2,6-Dichloranilin | 29 % 212-213°C | Ethanol/ Wasser |
| 59 | 2,3-Dihydro-3,3-dimethyl-N-(3,5-dichlorphenyl)-2-oxo-(1H)-indol-6-carboxamid aus 3,5-Dichloranilin | 60 % 261-265°C | Ethanol/ Wasser |

| | Bezeichnung | Ausbeute Fp. | umkrist. aus |
|---|---|---|---|
| 60 | 2,3-Dihydro-3,3-dimethyl-N-(8-chinolinyl)-2-oxo-(1H)-indol-6-carboxamid aus 8-Chinolinamin | 59 % 269-272°C | Ethanol |
| 61 | 2,3-Dihydro-3,3-dimethyl-N-(3,4-dichlorphenyl)-2-oxo-(1H)-indol-6-carboxamid aus 3,4-Dichloranilin | 49 % 302-304°C | Isopropanol |

Analog dem Beispiel 30 stellte man durch Umsetzung der 2,3-Dihydro-3,3-dimethyl-2-oxo-(1H)-indol-5-carbonsäure mit den angegebenen Anilinen die folgenden Beispielverbindungen her:

| | Bezeichnung | Ausbeute Fp. | umkrist. aus |
|---|---|---|---|
| 62 | 2,3-Dihydro-3,3-dimethyl-N-phenyl-2-oxo-(1H)-indol-5-carboxamid aus Anilin | 62 % 283-285 °C | Essigester |
| 63 | 2,3-Dihydro-3,3-dimethyl-N-(4-fluorphenyl)-2-oxo-(1H)-indol-5-carboxamid aus 4-Fluoranilin | 58 % 276-278 °C | Ethanol |

Beispiel 64

2,3-Dihydro-3,3-dimethyl-1-ethyl-N-(4-pyridinyl)-2-oxo-(1H)-indol-6-carboxamid

Analog dem Beispiel 35 erhielt man durch Umsetzung mit 4-Pyridinamin 60 % der Titelverbindung mit dem Fp. 209-211 °C nach Umkristallisation aus Ethanol/Wasser.

Beispiel 65

2,3-Dihydro-3,3-dimethyl-N-(3-aminophenyl)-2-oxo-(1H)-indol-6-carboxamid

Analog dem Beispiel 29 erhielt man durch Hydrierung der Beispielverbindung 57 die Titelverbindung in 95 % Ausbeute mit dem Fp. 244-245 °C nach Umkristallisation aus Methanol.

Beispiel 66

2,3-Dihydro-3,3-dimethyl-N-(4-acetaminophenyl)-2-oxo-(1H)-indol-6-carboxamid

0.83 g (2.4 mmol) 2,3-Dihydro-3,3-dimethyl-N-(4-aminophenyl)-2-oxo-(1H)-indol-6-carboxamid (aus Beispiel 29) rührte war in 5 ml 2N Essigsäure und 5 ml (53.4 mmol) Acetanhydrid 3 h bei 40 °C. Man entfernte das Lösungsmittel i. Vak. und reinigte den Rückstand säulenchromatographisch (Silicagel; Isohexan: Essigester: Methanol = 5 : 4 : 0.5) und erhielt 0.6 g (63 %) der Titelverbindung mit dem Fp. 294-295 °C.

Beispiel 67

2,3-Dihydro-3,3-dimethyl-N-(3-acetaminophenyl)-2-oxo-(1H)-indol-6-carboxamid

erhielt man in 73 % Ausbeute analog Beispiel 66 aus der in Beispiel 65 hergestellten Verbindung Fp. 311-313 °C.

Beispiel 68

2,3-Dihydro-3,3-dimethyl-(4-butylphenyl)-2-oxo-(1H)-indol-6-carboxamid

mit dem Fp. 208-210 °C erhielt man analog dem Beispiel 1 durch Umsetzung mit 4-Butylanilin.

Beispiel 69

2,3-Dihydro-3,3-dimethyl-(4-tert.butylphenyl)-2-oxo-(1H)-indol-6-carboxamid

mit dem Fp. 260-263 °C erhielt man in 78 % Ausbeute analog dem Beispiel 1 durch Umsetzung mit 4-tert. Butylanilin.

Beispiel 70

2,3-Dihydro-3,3-dimethyl-(4-octylphenyl)-2-oxo-(1H)-indol-6-carboxamid

mit dem Fp. 178-180 °C erhielt man in 47 % Ausbeute analog dem Beispiel 1 durch Umsetzung mit 4-n-Octylanilin.

Beispiel 71

2,3-Dihydro-3,3-dimethyl-N-(4-methoxycarbonyl-phenyl)-2-oxo-(1H)-indol-6-carboxamid

mit dem Fp. 258-260 °C erhielt man in 50 % Ausbeute analog dem Beispiel 1 durch Umsetzung mit 4-Aminobenzosäuremethylester.

Beispiel 72

2,3-Dihydro-3,3-dimethyl-N-(3-ethoxycarbonylpropyloxy-phenyl)-2-oxo-(1H)-indol-6-carboxamid

mit dem Fp. 96 - 98 °C erhielt man in 48 % Ausbeute analog dem Beispiel 1 durch Umsetzung mit 3-(3-Aminophenyloxy)butansäure-ethylester.

Beispiel 73

2′, 3′-Dihydro-N-phenyl-2′-oxo-spiro[cyclopentan-1,3′-(1′H)-indol]-6′-carboxamid

mit dem Fp. 249 °C erhielt man in 40 % Ausbeute analog dem Beispiel 48 durch Umsetzung mit Anilin.

## Patentansprüche

**1.** Verbindungen der allgemeinen Formel I

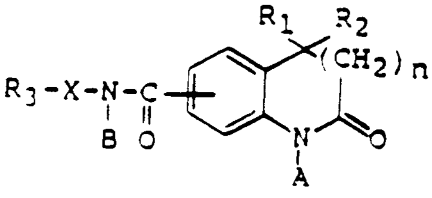

(I)

in der

A und B gleich oder verschieden sein können und jeweils ein Wasserstoffatom, eine $C_1$-$C_6$-Alkyl-, $C_2$-$C_6$-Alkenyl-, $C_2$-$C_6$-Alkinyl-, Benzyl- oder eine $C_3$-$C_7$-Cycloalkylgruppe bedeuten,

$R_1$ ein Wasserstoffatom, eine $C_1$-$C_6$-Alkyl-, $C_2$-$C_6$-Alkenyl-, oder $C_3$-$C_7$-Cycloalkylgruppe

$R_2$ ein Wasserstoffatom, eine $C_1$-$C_6$-Alkyl-, $C_2$-$C_6$-Al-kenyl-, $C_3$-$C_7$-Cycloalkyl-, $C_1$-$C_6$-Alkyl-

carbonyl-, $C_1$-$C_6$-Alkoxycarbonyl-, Aminocarbonyl- oder Hydrazinocarbonylgruppe bedeutet, oder $R_1$ und $R_2$ zusammen mit dem C-Atom, an das sie gebunden sind, einen $C_3$-$C_7$-Cycloalkylring bilden,

n die Werte 0 oder 1 annehmen kann,

X eine Bindung oder eine $C_1$-$C_6$-Alkylengruppe bedeutet,

$R_3$ einen aromatischen heterocyclischen Fünf- oder Sechsring mit 1 bis 4 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können und die Sechsringe gewünschtenfalls durch eine Pyridinyloxy- oder Phenyloxygruppe substituiert sein können oder die Fünf- oder Sechsringe mit einem Phenylring oder einem aromatischen Fünf- oder Sechsring mit 1 bis 4 Heteroatomen zu einem Bicyclus kondensiert sein können und gewünschtenfalls die Fünf- oder Sechsringe, die Bicyclen, die Pyridinyloxy- und die Phenyloxygruppe durch eine oder mehrere $C_1$-$C_6$-Alkyl-,$C_1$-$C_6$-Alkoxy-, $C_2$-$C_6$-Alkenyloxy-, $C_1$-$C_6$-Alkoxycarbonyl-, Carboxyl-, $C_1$-$C_6$-Alkylmercapto-, Hydroxy-, Nitro-, Amino-, Halogen-oder Cyangruppen substituiert sein können, oder

$R_3$ einen Phenylring der allgemeinen Formel II bedeutet,

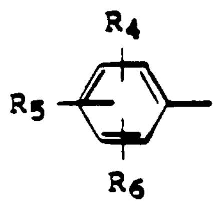

(II)

wobei $R_4$, $R_5$, $R_6$ gleich oder verschieden sein können und jeweils Wasserstoff, eine Imidazolylgruppe, eine gewünschtenfalls mit $C_1$-$C_6$-Alkylgruppen substituierte Oxopyridazinylgruppe, die gewünschtenfalls hydriert sein kann, eine $C_1$-$C_6$-Alkansulfonyloxy-, Trifluormethansulfonyloxy-, Phenylsulfonylamino-, $C_1$-$C_6$-Alkansulfonylamino-, Trifluormethansulfonylamino-, N-$C_1$-$C_6$-Alkylalkansulfonylamino-, N-$C_1$-$C_6$-Alkyltrifluormethansulfonylamino-, $C_1$-$C_6$-Alkylsulfenylmethyl-, $C_1$-$C_6$-Alkylsulfinylmethyl- oder $C_1$-$C_6$-Alkylsulfonylmethylgruppe, eine durch eine Hydroxy-, $C_1$-$C_6$-Alkoxy-, $C_1$-$C_6$-Alkylamino- oder Di-$C_1$-$C_6$-alkylaminogruppe substituierte Carbonylgruppe, eine durch eine Amino-, $C_1$-$C_6$-Alkylamino-, Di-$C_1$-$C_6$-alkylamino-, Piperidino- oder Morpholinogruppe substituierte Sulfonylgruppe, eine $C_1$-$C_6$-Alkylcarbonylamino-, Aminocarbonylamino- oder $C_1$-$C_6$-Alkylaminocarbonylaminogruppe, eine $C_1$-$C_6$-Alkylmermercapto-, $C_1$-$C_6$-Alkylsulfinyl- oder $C_1$-$C_6$-Alkylsulfonylgruppe, eine Nitro-, Halogen-, Amino-, Hydroxy-, $C_1$-$C_8$-Alkyl-, $C_1$-$C_6$-Alkoxy-, Pyridinyloxy-, $C_2$-$C_6$-Alkenyloxy-, $C_2$-$C_6$-Alkinyloxy-, Cyan-$C_1$-$C_6$-alkyloxy-, Carboxy-$C_1$-$C_6$-alkyloxy-, $C_1$-$C_6$-Alkoxycarbonyl-$C_1$-$C_6$-alkyloxy-, Di-$C_1$-$C_6$-alkylamino-, Trifluormethyl- oder Cyangruppe sein können oder $R_3$ einen Naphthyl-, Tetrahydronaphthyl-, Biphenyl-, Methylendioxyphenyl- oder Ethylendioxyphenylrest bedeutet,
mit der Maßangabe, daß für den Fall, daß n die Zahl 1 und $R_1$ und $R_2$ ein Wasserstoffatom bedeuten, X keine $C_1$-$C_6$-Alkylengruppe sein kann,
deren Tautomere, optisch aktive Formen oder physiologisch verträgliche Salze organischer oder anorganischer Säuren.

**2.** Verbindungen gemäß Anspruch 1, dadurch gekennzeichnet, daß A ein Wasserstoffatom, eine Benzyl-, $C_1$-$C_6$-Alkyl- oder $C_2$-$C_4$-Alkenylgruppe und B ein Wasserstoffatom oder ein $C_1$-$C_6$-Alkylgruppe bedeutet.

**3.** Verbindungen gemäß Anspruch 1 oder 2, dadurch gekennzeichnet, daß $R_1$ und $R_2$ jeweils eine $C_1$-$C_4$-Alkylgruppe oder $R_1$ und $R_2$ gemeinsam einen $C_5$-$C_6$-Cycloalkylring darstellen.

**4.** Verbindungen gemäß einem der Ansprüche 1 bis 3, dadurch gekennzeichnet, daß $R_3$ einen Pyrrolyl-, Furanyl-, Thiophenyl-, Pyrazolyl-, Imidazolyl-, Thiazolyl-, Tetrazolyl-, Isothiazolyl-, Oxazolyl-, Isoxazolyl-, Triazolyl-, Thiadiazolyl-, Oxadiazolyl-, Pyrazinyl-, N,N'-Dioxy-pyrazinyl-, Pyrimidinyl-, N,N'-Dioxypyrimidinyl-, Pyridazinyl-, Pyridazinyl-, Oxazinyl-, Thiazinyl-, Triazinyl-, Tetrazinyl-, Pyridinyl- oder N-Oxypyridinylrest, oder $R_3$ einen Indolyl-, Indazolyl-, Benzimidazolyl-, Chinolinyl-, Isochinolinyl-, Cinnolinyl-, Phthalazinyl-, Chinozolinyl-, Chinoxalinyl-, Benzofuranyl-, Benzothiophenyl-, Benzoxazolyl-, Benzisoxazolyl-, Benzothiazolyl-, Benzoisothiazolyl-, Benzotriazolyl- oder Benzothiadiazolylrest, oder $R_3$ einen Naphthylridinyl-, Pteridinyl-, Purinyl-, Indolizinyl-, Thiopheno[2,3-b]pyrazinyl-, Triazolo[4,3-a]pyridinyl- oder Imidazo(1,2-a]-pyridinylrest bedeutet.

**5.** Verbindungen gemäß einem der Ansprüche 1 bis 3, dadurch gekennzeichnet, daß $R_3$ einen Phenylrest bedeutet, der unsubstituiert oder durch $C_1$-$C_4$-Alkylsulfonyloxy-, Trifluormethansulfonyloxy-, $C_1$-$C_4$-Alkylsulfonylamino-, Trifluormethansulfonylamino-, $C_1$-$C_4$-Alkylmercapto-, $C_1$-$C_4$-Alkylsulfinyl-, $C_1$-$C_4$-Alkylsulfonyl-, Hydroxy-, $C_1$-$C_8$-Alkyl-, $C_1$-$C_4$-Alkoxy-, $C_2$-$C_4$-Alkinyloxy-, Trifluormethyl-, Imidazolyl-, Pyridinyloxy-, 4,5-dihydro-4-$C_1$-$C_4$-Alkyl-6-oxo-(1H)-Pyridazinyl-, $C_2$-$C_4$-Alkenyloxy-, $C_1$-$C_4$-Alkoxycarbonyl-$C_1$-$C_4$-alkyloxy-, Nitro-, Amino-, $C_1$-$C_4$-Alkyl-carbonylamino-, $C_1$-$C_4$-Alkoxy-carbonyl-, Chlor-, Fluor-, Cyano- oder Di-$C_1$-$C_4$-Alkylaminogruppen substituiert sein kann, oder $R_3$ einen Methylendioxyphenyl-, Ethylendioxyphenyl- Naphthyl-, Tetrahydronaphthyl- oder Biphenylrest bedeutet.

**6.** Verbindungen gemäß einem der Ansprüche 1-5, dadurch gekennzeichnet, daß für den Fall, daß n die Zahl 0 bedeutet, die $R_3$-X-N(B)-CO-Gruppe in 5- oder 6-Stellung des Oxindolrings steht, oder für den Fall, daß n die Zahl 1 bedeutet, die $R_3$-X-N(B)-CO-Gruppe in 7- oder 8-Stellung des Tetrahydrochinolinonrings steht.

**7.** Verbindungen gemäß einem der Ansprüche 1 bis 4 oder 6, dadurch gekennzeichnet, daß

$R_3$ den Pyrrolyl-, Furanyl-, Thiophenyl-, Pyrazolyl-, Imidazolyl-, Isothiazolyl-, Thiazolyl-, Oxazolyl-, Triazolyl-, Tetrazolyl-, Thiadiazolyl-, Isoxazolyl-, Oxadiazolyl-, Pyridinyl-, N-Oxypyridinyl-, Pyrazinyl-, N,N'-Dioxypyrazinyl-, Pyrimidinyl-, N,N'-Dioxypyrimidinyl-, Pyridazinyl-, Oxazinyl-, Thiazinyl-, Triazinyl-, Tetrazinyl-, Indolyl-, Benzimidazolyl-, Benzthiazolyl-, Indazolyl-, Chinolinyl, Pyridinyloxypyridinyl- oder Phenoxypyridinylrest darstellt sowie deren durch $C_1$-$C_4$-Alkyl-, $C_1$-$C_4$-Alkoxy, $C_2$-$C_4$-Alkenyloxy-, $C_1$-$C_4$-Alkylmercapto-, Chlor-, Amino- oder Hydroxy substituierten Derivate.

**8.** Verbindungen gemäß einem der Ansprüche 1 bis 7, dadurch gekennzeichnet, daß $R_1$ und $R_2$ jeweils eine Methylgruppe darstellen, und A und B ein Wasserstoffatom und X einen Valenzstrich bedeuten.

**9.** Verbindungen gemäß einem der Ansprüche 1-8, ausgewählt aus der folgenden Gruppe:
2,3-Dihydro-3,3-dimethyl-N-phenyl-2-oxo-(1H)-indol-6-carboxamid;
2,3-Dihydro-3,3-dimethyl-N-(3-trifluormethyl-phenyl)-2-oxo-indol-6-carboxamid;
2,3-Dihydro-3,3-dimethyl-N-(4-pyridinyl)-2-oxo-(1H)-indol-6-carboxamid;
2,3-Dihydro-3,3-dimethyl-N-[6-(3-pyridinyloxy)-3-pyridinyl]-2-oxo-(1H)-indol-6-carboxamid;
2,3-Dihydro-3,3-dimethyl-N-(4-hydroxy-2-methyl-phenyl)-2-oxo-(1H)-indol-6-carboxamid;
2,3-Dihydro-3,3-dimethyl-N-(4-fluorphenyl)-2-oxo-(1H)-indol-6-carboxamid;
2,3-Dihydro-3,3-dimethyl-N-(3-methoxyphenyl)-2-oxo-(1H)-indol-6-carboxamid.

**10.** Arzneimittel enthaltend mindestens eine Verbindung gemäß einem der Ansprüche 1-9 neben pharmazeutisch üblichen Hilfs- oder Trägerstoffen.

**11.** Verwendung von Verbindungen gemäß einem der Ansprüche 1-9 zur Herstellung von Arzneimitteln mit erythrozytenaggregations- oder thrombozytenaggregationshemmender Wirkung.

**12.** Verfahren zur Herstellung von Verbindungen der allgemeinen Formel I gemäß einem der Ansprüche 1-9, dadurch gekennzeichnet, daß man in an sich bekannter Weise ein Amin der allgemeinen Formel III

$R_3$-X-NH-B (III),

in der $R_3$, B und X die oben angegebenen Bedeutungen haben, mit einer Carbonsäure der allgemeinen Formel IV

in der A, $R_1$, $R_2$ und n die oben angegebenen Bedeutungen haben, oder mit einem reaktiven Derivat hiervon, zu Verbindungen der allgemeinen Formel I acyliert, oder gegebenenfalls die so hergestellten Verbindungen der allgemeinen Formel I nachträglich in andere Verbindungen der allgemeinen Formel I umwandelt.

**13.** Verbindungen der allgemeinen Formel IV

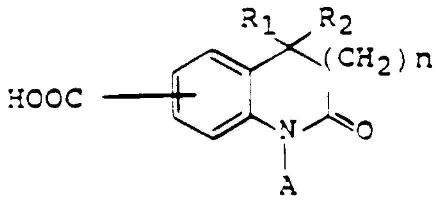

(IV)

in der

A ein Wasserstoffatom, eine $C_1$-$C_6$-Alkyl-, $C_2$-$C_6$-Alkenyl-, $C_2$-$C_6$-Alkinyl-, Benzyl- oder eine $C_3$-$C_7$-Cycloalkylgruppe bedeutet,

$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,

$R_2$ ein Wasserstoffatom, eine $C_1$-$C_6$-Alkyl-, $C_2$-$C_6$-Alkenyl, $C_3$-$C_7$-Cycloalkyl-, $C_1$-$C_6$-Alkylcarbonyl-, $C_1$-$C_6$-Alkoxycarbonyl-, Aminocarbonyl- oder Hydrazinocarbonylgruppe bedeutet oder $R_1$ und $R_2$ zusammen mit dem C-Atom, an das sie gebunden sind, einen $C_3$-$C_7$-Cycloalkylring bilden,

n die Werte 0 oder 1 annehmen kann, sowie

deren $C_1$-$C_6$-Alkylester, Anhydride oder Säurehalogenide,

mit Ausnahme der Verbindungen 1,2,3,4-Tetrahydro-2-oxo-7-chinolin-carbonsäure, 2,3-Dihydro-2-oxo-1H-indol-4-carbonsäure, 2,3-Dihydro-2-oxo-1H-indol-5-carbonsäure, 1-Ethyl-2,3 dihydro-2-oxo-(1H)-indol-5-carbonsäure, 1',2'-Dihydro-2'-oxo-spiro[cyclo-propan-1,3'-(3H)-indol]-6'-carbonsäure, 2,3-Dihydro-2-oxo-(1H)-indol-7-carbonsäure, 2,3-Dihydro-3,3-dimethyl-2-oxo-1H-indol-5-carbonsäure und 2,3-Dihydro-2-oxo-1H-indol-6-carbonsäure.

**14.** Verfahren zur Herstellung von Verbindungen der allgemeinen Formel IV gemäß Anspruch 13, dadurch gekennzeichnet, daß man

a) Verbindungen der allgemeinen Formel V

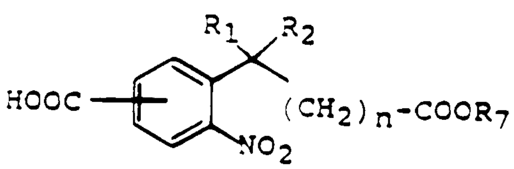

(V)

in der $R_1$, $R_2$ und n die oben angegebenen Bedeutungen haben, und $R_7$ ein Wasserstoffatom oder eine $C_1$-$C_4$-Alkylgruppe darstellt, reduziert, wobei unter Ringschluß Verbindungen der allgemeinen Formel IV resultieren, oder

b) Verbindungen der allgemeinen Formel VII

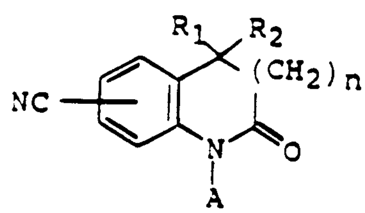

(VII)

in der A, $R_1$, $R_2$ und n die oben angegebenen Bedeutungen haben, verseift,

oder die so hergestellten Verbindungen der allgemeinen Formel IV nachträglich in andere Verbindungen der allgemeinen Formel IV umwandelt.

**15.** Verbindungen der allgemeinen Formel VII

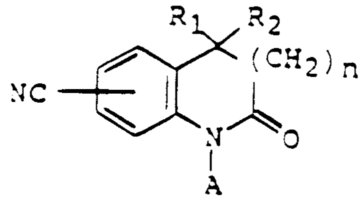

(VII)

in der

A ein Wasserstoffatom, eine $C_1$-$C_6$-Alkyl-, $C_2$-$C_6$-Alkenyl-, $C_2$-$C_6$-Alkinyl-, Benzyl- oder eine $C_3$-$C_7$-Cycloalkylgruppe bedeutet,

$R_1$ ein Wasserstoffatom, eine $C_1$-$C_6$-Alkyl-, $C_2$-$C_6$-Alkenyl- oder eine $C_3$-$C_7$-Cycloalkylgruppe bedeutet,

$R_2$ ein Wasserstoffatom, eine $C_1$-$C_6$-Alkyl-, $C_2$-$C_6$-Alkenyl, $C_3$-$C_7$-Cycloalkyl-, $C_1$-$C_6$-Alkylcarbonyl-, $C_1$-$C_6$-Alkoxycarbonyl-, Aminocarbonyl- oder Hydrazinocarbonylgruppe bedeutet oder $R_1$ und $R_2$ zusammen mit dem C-Atom, an das sie gebunden sind, einen $C_3$-$C_7$-Cycloalkylring bilden, und

n die Werte 0 oder 1 annehmen kann,

mit Ausnahme der Verbindung 2,3-Dihydro-2-oxo-(1H)-indol-5-carbonitril.

**16.** Verfahren zur Herstellung von Verbindungen der allgemeinen Formel VII gemäß Anspruch 15, dadurch gekennzeichnet, daß man Verbindungen der allgemeinen Formel (VIII)

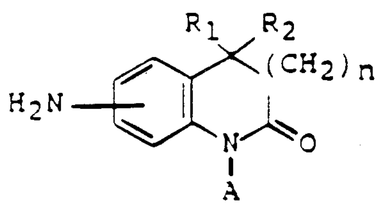

(VIII)

in der A, $R_1$, $R_2$ und n die oben genannten Bedeutungen haben, diazotiert und mit Cyaniden umsetzt (Sandmeyer-Reaktion).

**17.** Verwendung von Verbindungen gemäß Anspruch 13 oder 15 zur Herstellung von Verbindungen gemäß einem der Ansprüche 1-8.

## Claims

**1.** Compounds of the general formula I

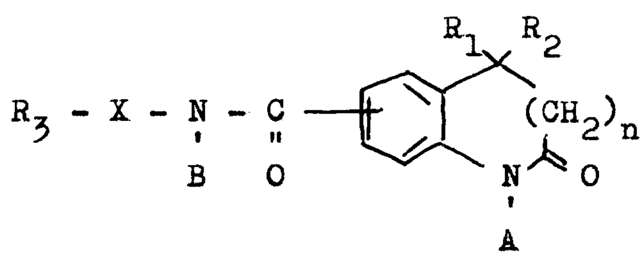

(I)

in which A and B can be the same or different and, in each case, signify a hydrogen atom, a $C_1$-$C_6$-alkyl, $C_2$-$C_6$-alkenyl, $C_2$-$C_6$-alkynyl, benzyl or a $C_3$-$C_7$-cycloalkyl group, $R_1$ signifies a hydrogen atom, a $C_1$-$C_6$-alkyl, $C_2$-$C_6$-alkenyl or $C_3$-$C_7$-cycloalkyl group, $R_2$ a hydrogen atom, a $C_1$-$C_6$-alkyl, $C_2$-$C_6$-alkenyl, $C_3$-$C_7$-cycloalkyl, $C_1$-$C_6$-alkylcarbonyl, $C_1$-$C_6$-alkoxycarbonyl, aminocarbonyl or hydrazinocar-

bonyl group or $R_1$ and $R_2$, together with the C-atom to which they are attached, form a $C_3$-$C_7$-cycloalkyl ring, n can have the values 0 or 1, X signifies a bond or a $C_1$-$C_6$-alkylene groups $R_3$ represents an aromatic heterocylic five- or six-membered ring with 1 to 4 heteroatoms, whereby the heteroatoms can be the same or different and signify oxygen, sulphur or nitrogen and, if desired, can carry an oxygen atom on one or more nitrogen atoms and the six-membered ring can, if desired, be substituted by a pyridinyloxy or phenyloxy group or the five- or six-membered ring can be condensed with a phenyl ring or an arometic five- or six-membered ring with 1 to 4 heteroatoms to give a bicycle and, if desired, the five- or six-membered rings, the bicycles, the pridinyloxy and the phenyloxy group can be substituted by one or more $C_1$-$C_6$-alkyl, $C_1$-$C_6$-alkoxy, $C_2$-$C_6$-alkenyloxy, $C_1$-$C_6$-alkoxycarbonyl, carboxyl, $C_1$-$C_6$-alkylmercapto, hydroxyl, nitro, amino, halogen or cyano groups or $R_3$ signifies a phenyl ring of the general formula II

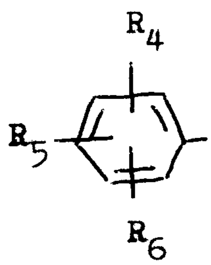

(II)

whereby $R_4$, $R_5$, $R_6$ can be the same or different and, in each case, signify hydrogen, an imidazolyl radical, an oxopyridazinyl group,substituted, if desired, with $C_1$-$C_6$-alkyl groups,which, if desired, can be hydrogenated, a $C_1$-$C_6$-alkanesulphonyloxy, trifluoromethanesulphonyloxy, phenylsulphonylamino, $C_1$-$C_6$-alkanesulphonylamino, trifluoromethanesulphonylamino, N-$C_1$-$C_6$-alkyl-alkanesulphonylamino, N-$C_1$-$C_6$-alkyltrifluoromethanesulphonylamino, $C_1$-$C_6$-alkylsulphenylmethyl, $C_1$-$C_6$-alkylsulphinylmethyl or $C_1$-$C_6$-alkylsulphonylmethyl group, a carbonyl group substituted by a hydroxyl, $C_1$-$C_6$-alkoxy, $C_1$-$C_6$-alkylamino or di-$C_1$-$C_6$-alkylamino group, a sulphinyl group substituted by an amino, $C_1$-$C_6$-alkylamino, di-$C_1$-$C_6$-alkylamino, piperidino or morpholino group, a $C_1$-$C_6$-alkylcarbonylamino, aminocarbonylamino or $C_1$-$C_6$-alkylaminocarbonylamino group, a $C_1$-$C_6$-alkylmercapto, $C_1$-$C_6$-alkylsulphinyl or $C_1$-$C_6$-alkylsulphonyl group, a nitro, halogen, amino, hydroxyl, $C_1$-$C_8$-alkyl, $C_1$-$C_6$-alkoxy, pyridinyloxy, $C_2$-$C_6$-alkenyloxy, $C_2$-$C_6$-alkynyloxy, cyano-$C_1$-$C_6$-alkyloxy, carboxy-$C_1$-$C_6$-alkoxy, $C_1$-$C_6$-alkoxycarbonyl-$C_1$-$C_6$-alkyloxy, di-$C_1$-$C_6$-alkylamino, trifluoromethyl or cyano group or $R_3$ signifies a naphthyl, tetrahydronaphthyl, biphenyl, methylenedioxyphenyl or ethylenedioxyphenyl radical, with the proviso that, for the case that n signifies the number 1 and $R_1$ and $R_2$ a hydrogen atom, X cannot be a $C_1$-$C_6$-alkylene group, their tautomers, optically-active forms or physiologically compatible salts of organic or inorganic acids.

**2.** Compounds according to claim 1, characterised in that A signifies a hydrogen atom, a benzyl, $C_1$-$C_6$-alkylor $C_2$-$C_4$-alkenyl group and B is a hydrogen atom or a $C_1$-$C_6$-alkyl group.

**3.** Compounds according to claim 1 or 2, characterised in that $R_1$ and $R_2$ each represent a $C_1$-$C_4$-alkyl group or $R_1$ and $R_2$ together represent a $C_5$-$C_6$-cycloalkyl ring.

**4.** Compounds according to one of claims 1 to 3, characterised in that $R_3$ signifies a pyrrolyl, furanyl, thiophenyl, pyrazolyl, imidazolyl, thiazolyl, tetrazolyl, isothiazolyl, oxazolyl, isoxazolyl, triazolyl, thiadiazolyl, oxadiazolyl, pyrazinyl, N,N'-dioxypyrazinyl, pyrimidinyl, N,N'-dioxypyrimidinyl, pyridazinyl, oxazinyl, thiazinyl, triazinyl, tetrazinyl, pyridinyl or N-oxypyridinyl radical or $R_3$ an indolyl, indazolyl, benzimidazolyl, quinolinyl, isoquinolinyl, cinnolinyl, phthalazinyl, quinazolinyl, quinoxalinyl, benzofuranyl, benzothiophenyl, benzoxazolyl, benzisoxazolyl, benzothiazolyl, benzoisothiazolyl, benzotriazolyl or benzothiadiazolyl radical or $R_3$ a naphthylridinyl, pteridinyl, purinyl, indolizinyl, thiophene [2,3-b] pyrazinyl, triazolo-[4,3-a] pyridinyl or imidazo [1,2-a] pyridinyl radical.

**5.** Compounds according to one of claims 1 to 3, characterised in that $R_3$ signifies a phenyl radical which can be unsubstituted or substituted by $C_1$-$C_4$-alkylsulphonyloxy, trifluoromethonesulphonyloxy, $C_1$-$C_4$-alkylsulphonylamino, trifluoromethanesulphonylamino, $C_1$-$C_4$-alkylmercapto, $C_1$-$C_4$-alkylsulphinyl, $C_1$-$C_4$-alkylsulphonyl, hydroxyl, $C_1$-$C_8$-alkyl, $C_1$-$C_4$-alkoxy, $C_2$-$C_4$-alkynyloxy, trifluoromethyl, imidazolyl,

pyridinyloxy, 4,5-dihydro-4-$C_1$-$C_4$-alkyl-6-oxo-(1H)-pyridazinyl, $C_2$-$C_4$-alkenyloxy, $C_1$-$C_4$-alkoxycarbonyl-$C_1$-$C_4$-alkoxy, nitro, amino, $C_1$-$C_4$-alkylcarbonylamino, $C_1$-$C_4$-alkoxycarbonyl, chlorine, fluorine, cyano or di-$C_1$-$C_4$-alkylamino groups or $R_3$ a methylenedioxyphenyl, ethylenedioxyphenyl, naphthyl, tetrahydronaphthyl or biphenyl radical.

**6.** Compounds according to one of claims 1 - 5, characterised in that, for the case that n signifies the number 0, the $R_3$-X-N(B)-CO- group stands in the 5- or 6-position of the oxindole ring or, for the case that n signifies the number 2, the $R_3$-X-N(B)-CO- group stands in the 7- or 8-position of the tetrahydroquinoline ring.

**7.** Compounds according to one of claims 1 to 4 or 6, characterised in that $R_3$ represents the pyrrolyl, furanyl, thiophenyl, pyrazolyl, imidazolyl, isothiazolyl, thiazolyl, oxazolyl, triazolyl, tetrazolyl, thiadiazolyl, isoxazolyl, oxadiazolyl, pyridinyl, N-oxypyridinyl, pyrazinyl, N,N'-dioxypyrazinyl, pyrimidinyl, N,N'- dioxypyrimidinyl, pyridazinyl, oxazinyl, thiazinyl, triazinyl, tetrazinyl, indolyl, benzimidazolyl, benzthiazolyl, indazolyl, quinolinyl, pyridinyloxypyridinyl or phenoxypyridinyl radical, as well as their derivatives substituted by $C_1$-$C_4$-alkyl, $C_1$-$C_4$-alkoxy, $C_2$-$C_4$-alkenyloxy, $C_1$-$C_4$-alkylmercapto, chlorine, amino or hydroxyl,

**8.** Compounds according to one of claims 1 to 7, characterised in that $R_1$, and $R_2$ each represent a methyl group and A and B signify a hydrogen atom and X a valency bond.

**9.** Compounds according to one of claims 1 - 8 selected from the following group:
2,3-dihydro-3,3-dimethyl-N-phenyl-2-oxo-(1H)-indole6-carboxamide;
2,3-dihydro-3,3-dimethyl-N-(3-trifluoromethylphenyl)-2-oxoindole-6-carboxamide;
2,3-dihydro-3,3-dimethyl-N-(4-pyridinyl)-2-oxo-(1H)-indole-6-carboxamide;
2,3-dihydro-3,3-dimethyl-N-[6-(3-pyridinyloxy)-3-pyridinyl]-2-oxo-(1H)-indole-6-carboxamide;
2, 3-dihydro-3,3-dimethyl-N-(4-hydroxy-2-methylphenyl)-2-oxo-(1H)-indole-6-carboxamide;
2,3-dihydro-3,3-dimethyl-N-(4-fluorophenyl)-2-oxo-(1H)-indole-6-carboxamide;
2,3-dihydro-3,3-dimethyl-N-(3-methoxyphenyl)-2-oxo-(1H)-indole-6-carboxamide.

**10.** Medicaments containing at least one compound according to one of claims 1 - 9, besides pharmaceutically usual adjuvant or carrier materials.

**11.** Use of compounds according to one of claims 1 - 9 for the preparation of medicaments with erythrocyte aggregation- and thrombocyte aggregation-inhibiting action.

**12.** Process for the preparation of compounds of the general formula according to one of claims 1 - 9, characterised in that, in per se known manner, one acylates an amine of the general formula III

$R_3$-X-NH-B (III)

in which $R_3$, B and X have the above-given meanings, with a carboxylic acid of the general formula IV

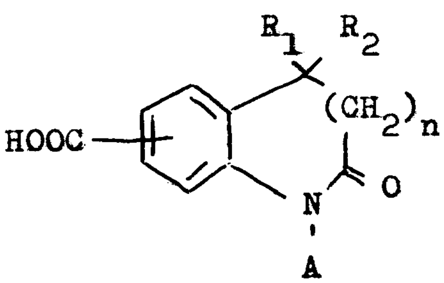

(IV)

in which A, $R_1$, $R_2$ and n have the above-given meanings, or with a reactive derivative hereof to give compounds of the general formula I or possibly subsequently converts the so-prepared compounds of the general formula I into other compounds of the general formula I.

**13.** Compounds of the general formula IV

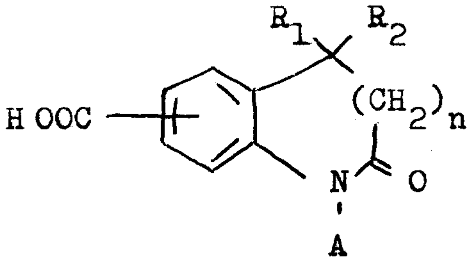

(IV)

in which A signifies a hydrogen atom, a $C_1$-$C_6$-alkyl, $C_2$-$C_6$-alkenyl, $C_2$-$C_6$-alkynyl, benzyl or a $C_3$-$C_7$-cycloalkyl group, $R_1$ signifies a hydrogen atom, a $C_1$-$C_6$-alkyl, $C_2$-$C_6$-alkenyl or a $C_3$-$C_7$-cycloalkyl group, $R_2$ signifies a hydrogen atom, a $C_1$-$C_6$-alkyl, $C_2$-$C_6$-alkenyl, $C_3$-$C_7$-cycloalkyl, $C_1$-$C_6$-alkylcarbonyl, $C_1$-$C_6$-alkoxycarbonyl, aminocarbonyl or hydrazinocarbonyl group or $R_1$ and $R_2$, together with the C-atom to which they are attached, form a $C_3$-$C_7$-cycloalkyl ring, n can assume the values 0 or 1, as well as their $C_1$-$C_6$-alkyl eaters, anhydrides or acid halides, with the exception of the compounds 1,2,3,4-tetrahydro-2-oxo-7-quinolinecarboxylic acid, 2,3-dihydro-2-oxo-1H-indole-4-carboxylic acid, 2,3-dihydro-2-oxo-1H-indole-5-carboxylic acid, 1-ethyl-2,3-dihydro-2-oxo-(1H)-indole-5-carboxylic acid, 1',2'-dihydro-2'-oxo-spiro-[cyclopropane-1,3'-(3H)-indole]-6)carboxylic acid, 2,3-dihydro-2-oxo-(1H)-indole-7-carboxylic acid, 2,3-dihydro-3,3-dimethyl-2-oxo-1H-indole-5-carboxylic acid and 2,3-dihydro-2-oxo-1H-indole-6-carboxylic acid.

**14.** Process for the preparation of compounds of the general formula IV according to claim 13, characterised in that one

a) reduces compounds of the general formula V

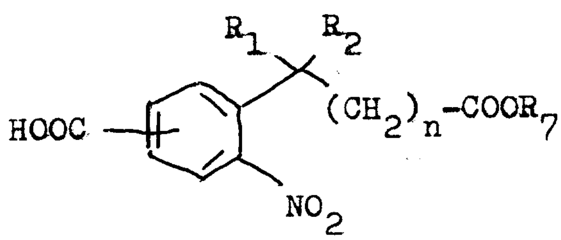

(V)

in which $R_1$, $R_2$ and n have the above-given meanings and $R_7$ represents a hydrogen atom or a $C_1$-$C_4$-alkyl group, whereby, with ring closure, compounds of the general formula IV result; or

b) saponifies compounds of the general formula VII

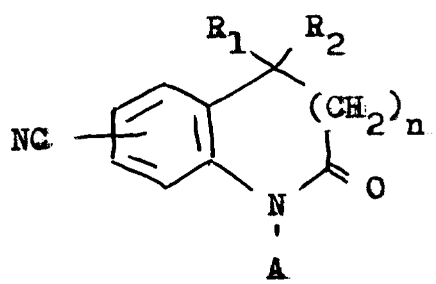

(VII)

in which A, $R_1$, $R_2$ and n have the above-given meanings, or subsequently converts the so-prepared compounds of the general formula IV into other compounds of the general formula IV.

**15.** Compounds of the general formula VII

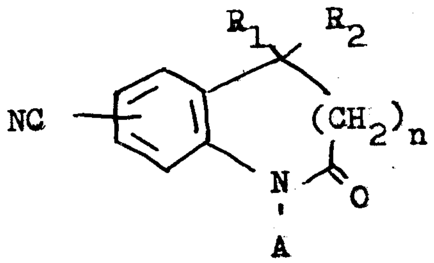

(VII)

in which A signifies a hydrogen atom, a $C_1$-$C_6$-alkyl, $C_2$-$C_6$-alkenyl, $C_2$-$C_6$-alkynyl, benzyl or a $C_3$-$C_7$-cycloalkyl group, $R_1$ signifies a hydrogen atom, a $C_1$-$C_6$-alkyl, $C_2$-$C_6$-alkenyl or a $C_3$-$C_7$-cycloalkyl group, $R_2$ signifies a hydrogen atom, a $C_1$-$C_6$-alkyl, $C_2$-$C_6$-alkenyl,, $C_3$-$C_7$-cycloalkyl, $C_1$-$C_6$-alkylcarbonyl, $C_1$-$C_6$-alkoxycarbonyl, aminocarbonyl or hydrazinocarbonyl group or $R_1$ and $R_2$, together with the C-atom to which they are attached, form a $C_3$-$C_7$-cycloalkyl ring and n can assume the values 0 or 1, with the exception of the compound 2,3-dihydro-2-oxo-(1H)-indole-5-carbonitrile.

**16.** Process for the preparation of compounds of the general formula VII according to claim 15, characterised in that one diazotises compounds of the general formula (VIII)

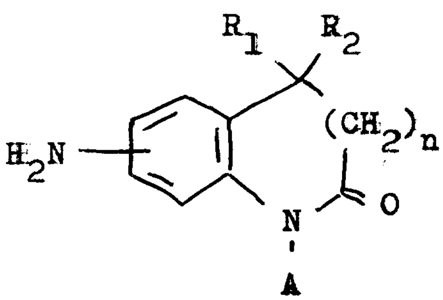

(VIII)

in which A, $R_1$, $R_2$ and n have the above-given meanings, and reacts with cyanides (Sandmeyer reaction)

**17.** Use of compounds according to claim 13 or 15 for the preparation of compounds according to one of claims 1 to 8.

**Revendications**

**1.** Composés de formule générale I

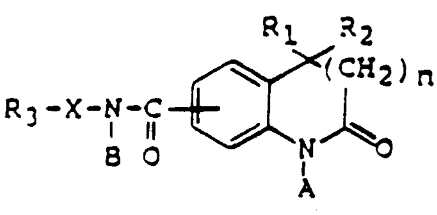

(I)

dans laquelle

A et B peuvent être identiques ou différents et représentent chacun un atome d'hydrogène, un groupe alkyl($C_1$-$C_6$), alcényle($C_2$-$C_6$), alcynyle($C_2$-$C_6$), benzyle ou cycloalkyle($C_3$-$C_7$),

$R_1$ représente un atome d'hydrogène, un groupe alkyle($C_1$-$C_6$), alcényle($C_2$-$C_6$) ou cycloalkyle($C_3$-$C_7$),

$R_2$ représente un atome d'hydrogène, un groupe alkyle($C_1$-$C_6$), alcényle($C_2$-$C_6$), cycloalkyle-($C_3$-$C_7$), alkyl($C_1$-$C_6$)carbonyle, alcoxy($C_1$-$C_6$)-carbonyle, aminocarbonyle ou hydrazinocarbonyle, ou $R_1$ et $R_2$ forment, conjointement avec l'atome de C auquel ils sont liés, un cycle cycloalkyle($C_3$-$C_7$),

n peut prendre la valeur de 0 ou de 1,

X représente une liaison ou un groupe alkylène($C_1$-$C_6$),

$R_3$ représente un hétérocycle aromatique à cinq ou six chaînons comportant 1 à 4 hétéroatomes, les hétéroatomes pouvant être identiques ou différents et représentent l'oxygène, le soufre ou l'azote et éventuellement, un ou plusieurs atomes d'azote peuvent porter un atome d'oxygène, et le cycle à six chaînons peut être éventuellement substitué par un groupe pyridinyloxy ou phényloxy, ou bien le cycle à cinq ou six chaînons peut être condensé avec un noyau phényle ou avec un cycle aromatique à cinq ou six chaînons, comportant 1 à 4 hétéroatomes, pour donner un bicycle, et éventuellement, le cycle à cinq ou six chaînons, le bicycle, le groupe pyridinyloxy et le groupe phényloxy peuvent être substitués par un ou plusieurs groupes alkyl($C_1$-$C_6$), alcoxy($C_1$-$C_6$), alcényloxy($C_2$-$C_6$), alcoxy($C_1$-$C_6$)-carbonyle, carboxyle, alkyl($C_1$-$C_6$)-mercapto, hydroxy, nitro, amino, halogéno ou cyano, ou

$R_3$ représente un noyau phényle de formule générale II,

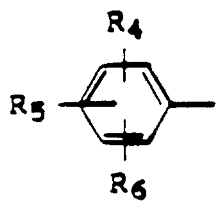

(II)

dans laquelle $R_4$, $R_5$, $R_6$ peuvent être identiques ou différents et représentent chacun un atome d'hydrogène, un groupe imidazolyle, un groupe oxopyridazinyle éventuellement substitué par un groupe alkyle($C_1$-$C_6$), qui peut être éventuellement hydrogéné, un groupe alcane($C_1$-$C_6$)-sulfonyloxy, trifluorométhane-sulfonyloxy, phénylsulfonylamino, alcane($C_1$-$C_6$)-sulfonylamino, trifluorométhanesulfonylamino, N-alkyl($C_1$-$C_6$)-trifluorométhanesulfonylamino, alkyl($C_1$-$C_6$)-sulfénylméthyle, alkyl($C_1$-$C_6$)-sulfinylméthyle ou alkyl($C_1$-$C_6$)-sulfonylméthyle, un groupe carbonyle substitué par un groupe hydroxy, alcoxy($C_1$-$C_6$),alkyl($C_1$-$C_6$)-amino ou dialkyl($C_1$-$C_6$)-amino, un groupe sulfonyle substitué par un groupe amino, alkyl($C_1$-$C_6$)-amino, dialkyl($C_1$-$C_6$)-amino, pipéridino ou morpholino, un groupe alkyl($C_1$-$C_6$)-carbonylamino, aminocarbonylamino ou alkyl($C_1$-$C_6$)-aminocarbonylamino, un groupe alkyl($C_1$-$C_6$)-mercapto, alkyl($C_1$-$C_6$)-sulfinyle ou alkyl($C_1$-$C_6$)-sulfonyle, un groupe nitro, halogéno, amino, hydroxy, alkyle($C_1$-$C_8$), alcoxy($C_1$-$C_6$), pyridinyloxy, alcényl($C_2$-$C_6$)-oxy, alcynyl($C_2$-$C_6$)-oxy, cyanoalkyl($C_1$-$C_6$)-oxy, carboxyalkyl($C_1$-$C_6$)-oxy, alcoxy($C_1$-$C_6$)-carbonylalkyl($C_1$-$C_6$)-oxy, dialkyl($C_1$-$C_6$)-amino, trifluorométhyle ou cyano, ou bien $R_3$ représente un groupe naphtyle, tétrahydronaphtyle, biphényle, méthylènedioxyphényle ou éthylènedioxyphényle,

étant entendu que dans le cas où n vaut 1 et $R_1$ et $R_2$ représentent un atome d'hydrogène, X ne peut représenter un groupe alkylène($C_1$-$C_6$),
leurs tautomères, leurs formes optiquement actives ou leurs sels physiologiquement acceptables formés avec des acides organiques ou inorganiques.

2. Composés selon la revendication 1, caractérisés en ce que A représente un atome d'hydrogène, un groupe benzyle, alkyle($C_1$-$C_6$) ou alcényle($C_2$-$C_4$) et B représente un atome d'hydrogène ou un groupe alkyle($C_1$-$C_6$).

3. Composés selon la revendication 1 ou 2, caractérisés en ce que $R_1$ et $R_2$ représentent chacun un groupe alkyle($C_1$-$C_4$) ou $R_1$ et $R_2$ représentent ensemble un cycle cycloalkyle($C_5$-$C_6$).

4. Composés selon l'une quelconque des revendications 1 à 3, caractérisés en ce que $R_3$ représente un groupe pyrrolyle, furanyle, thiophényle, pyrazolyle, imidazolyle, thiazolyle, tétrazolyle, isothiazolyle,

oxazolyle, isoxazolyle, triazolyle, thiadiazolyle, oxadiazolyle, pyrazinyle, N,N'-dioxy-pyrazinyle, pyrimidinyle, N,N'dioxy-pyrimidinyle pyridazinyle, pyridazinyle, oxazinyle, thiazinyle, triazinyle, tétrazinyle, pyridinyle ou N-oxypyridinyle, ou $R_3$ représente un groupe indolyle, indazolyle, benzimidazolyle, quinoléinyle, isoquinoléinyle, cinnolinyle, phtalazinyle, quinozolinyle, quinoxalinyle, benzofuranyle, benzothiophényle, benzoxazolyle, benzisoxazolyle, benzothiazolyle, benzoisothiazolyle, benzotriazolyle ou benzothiadiazolyle, ou $R_3$ représente un groupe naphtylpyridinyle, ptéridinyle, purinyle, indolizinyle, thiophéno[2,3-b]pyrazinyle, triazolo[4,3-a]pyridinyle ou imidazo[1,2-a]pyridinyle.

**5.** Composés selon l'une quelconque des revendications 1 à 3, caractérisés en ce que $R_3$ représente un reste phényle, qui peut être non substitué ou substitué par un groupe alkyl($C_1$-$C_4$)-sulfonyloxy, trifluorométhanesulfonyloxy, alkyl($C_1$-$C_4$)-sulfonylamino, trifluorométhanesulfonylamino, alkyl($C_1$-$C_4$)-mercapto, alkyl($C_1$-$C_4$)-sulfinyle, alkyl(-$C_1$-$C_4$)-sulfonyle, hydroxy, alkyle($C_1$-$C_8$), alcoxy($C_1$-$C_4$), alcynyl-($C_2$-$C_4$)oxy, trifluorométhyle, imidazolyle, pyridinyloxy, 4,5-dihydro-4-alkyl($C_1$-$C_4$)-6-oxo-(1H)-pyridazinyle, alcényl($C_2$-$C_4$)-oxy, alcoxy($C_1$-$C_4$)-carbonylalkyl($C_1$-$C_4$)-oxy, nitro, amino, alkyl($C_1$-$C_4$)-carbonylamino, alcoxy($C_1$-$C_4$)carbonyle, chloro, fluoro, cyano ou dialkyl($C_1$-$C_4$)-amino, ou bien $R_3$ représente un groupe méthylènedioxyphényle, éthylènedioxyphényle, naphtyle, tétrahydronaphtyle ou biphényle.

**6.** Composés selon l'une quelconque des revendications 1-5, caractérisés en ce que dans le cas où n vaut 0, le groupe $R_3$-X-N-(B)-CO se trouve en position 5 ou 6 du cycle oxindole, ou bien dans le cas où n vaut 1, le groupe $R_3$-X-N-(B)-CO se trouve en position 7 ou 8 du cycle tétrahydroquinoléinone.

**7.** Composés selon l'une quelconque des revendications 1 à 4 ou 6, caractérisés en ce que

$R_3$ représente un groupe pyrrolyle, furanyle, thiophényle, pyrazolyle, imidazolyle, isothiazolyle, thiazolyle, oxazolyle, triazolyle, tétrazolyle, thiadiazolyle, isoxazolyle, oxadiazolyle, pyridinyle, N-oxypyridinyle, pyrazinyle, N,N'-dioxypyrazinyle, pyrimidinyle, N,N'-dioxypyrimidinyle, pyridazinyle, oxazinyle, thiazinyle, triazinyle, tétrazinyle, indolyle, benzimidazolyle, benzothiazolyle, indazolyle, quinoléine, pyridinyloxypyridinyle ou phénoxypyridinyle, ainsi que leurs dérivés substitués par un groupe alkyle($C_1$-$C_4$), alcoxy($C_1$-$C_4$), alcényloxy($C_2$-$C_4$), alkyl($C_1$-$C_4$)-mercapto, chloro, amino ou hydroxy.

**8.** Composés selon l'une quelconque des revendications 1 à 7, caractérisés en ce que $R_1$ et $R_2$ représentent chacun un groupe méthyle, et A et B représentent un atome d'hydrogène et X une liaison.

**9.** Composés selon l'une quelconque des revendications 1 à 8, choisis dans le groupe suivant :

2,3-dihydro-3,3-diméthyl-N-phényl-2-oxo-(1H)-indol-6-carboxamide ;
2,3-dihydro-3,3-diméthyl-N-(3-trifluorométhyl-phényl)-2-oxo-indol-6-carboxamide ;
2,3-dihydro-3,3-diméthyl-N-(4-pyridinyl)-2-oxo-(1H)-indol-6-carboxamide ;
2,3-dihydro-3,3-diméthyl-N-[6-(3-pyridinyloxy)-3-pyridinyl]-2-oxo-(1H)-indol-6-carboxamide ;
2,3-dihydro-3,3-diméthyl-N-(4-hydroxy-2-méthyl-phényl)-2-oxo-(1H)-indol-6-carboxamide ;
2,3-dihydro-3,3-diméthyl-N-(4-fluorophényl)-2-oxo(1H)-indol-6-carboxamide ;
2,3-dihydro-3,3-diméthyl-N-(3-méthoxyphényl)-2-oxo-(1H)-indol-6-carboxamide.

**10.** Médicament contenant au moins un composé selon l'une quelconque des revendications 1-9, en plus d'adjuvants et véhicules pharmaceutiques usuels.

**11.** Utilisation de composés selon l'une quelconque des revendications 1-9, pour la préparation de médicaments ayant une activité inhibitrice de l'aggrégation des érythrocytes ou des thrombocytes.

**12.** Procédé de préparation des composés de formule générale I selon l'une quelconque des revendications 1-9, caractérisé en ce que, de manière connue en soi, une amine de formule générale III

$R_3$-X-NH-B (III),

dans laquelle $R_3$, B et X ont les significations mentionnées ci-dessus, est acylée à l'aide d'un acide carboxylique de formule générale IV

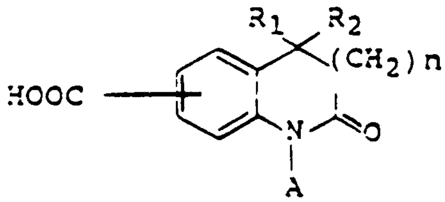

(IV)

dans laquelle A, $R_1$, $R_2$ et n ont les significations mentionnées ci-dessus, ou à l'aide d'un de ses dérivés réactifs, pour donner des composés de formule générale I, ou éventuellement, les composés de formule générale I ainsi obtenus sont transformés ultérieurement en d'autres composés de formule générale I.

**13.** Composés de formule générale IV

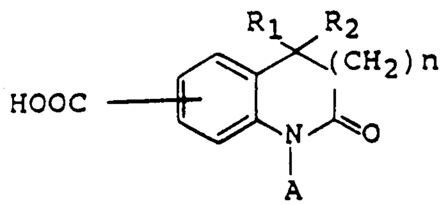

(IV)

dans laquelle

- A représente un atome d'hydrogène, un groupe alkyle($C_1$-$C_6$), alcényle-($C_2$-$C_6$), alcynyle-($C_2$-$C_6$), benzyle ou cycloalkyle-($C_3$-$C_7$),
- $R_1$ représente un atome d'hydrogène, un groupe alkyle($C_1$-$C_6$), alcényle($C_2$-$C_6$) ou cycloalkyle-($C_3$-$C_7$),
- $R_2$ représente un atome d'hydrogène, un groupe alkyle($C_1$-$C_6$), alcényle($C_2$-$C_6$), cycloalkyle($C_3$-$C_7$), alkyl($C_1$-$C_6$)-carbonyle, alcoxy($C_1$-$C_6$)-carbonyle, aminocarbonyle ou hydrazinocarbonyle, ou bien $R_1$ et $R_2$ forment, conjointement avec l'atome de C auquel ils sont liés, un cycle cycloalkyle-($C_3$-$C_7$),
- n vaut 0 ou 1,

ainsi que leurs esters d'alkyle($C_1$-$C_6$), leurs anhydrides ou leurs halogénures d'acide,

à l'exception des composés qui sont l'acide 1,2,3,4-tétrahydro-2-oxo-7-quinoléine-carboxylique, l'acide 2,3-dihydro-2-oxo-1H-indol-4-carboxylique, l'acide 2,3-dihydro-2-oxo-1H-indol-5-carboxylique, l'acide 1-éthyl-2,3-dihydro-2-oxo(1H)-indol-5-carboxylique, l'acide 1',2'-dihydro-2'-oxo-spiro[cyclo-propane-1,3'-(3H)-indol]-6'-carboxylique, l'acide 2,3-dihydro-2-oxo-(1H)indol-7-carboxylique, l'acide 2,3-dihydro-3,3-diméthyl-2-oxo-1H-indol-5-carboxylique et l'acide 2,3-dihydro-2-oxo-1H-indol-6-carboxylique.

**14.** Procédé de préparation des composés de formule générale IV selon la revendication 13, caractérisé en ce que

a) on réduit des composés de formule générale V

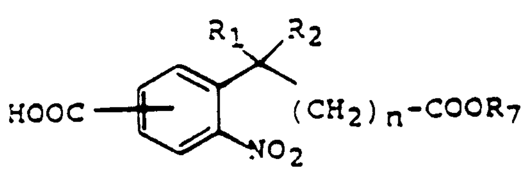

(V)

dans laquelle

$R_1$, $R_2$ et n ont les significations mentionnées ci-dessus, et $R_7$ représente un atome d'hydrogène ou un groupe alkyle($C_1$-$C_4$), et l'on obtient, avec fermeture du cycle, des composés de formule générale IV, ou bien

b) on saponifie des composés de formule générale VII

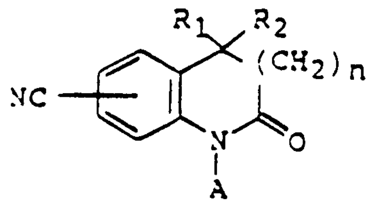

(VII)

dans laquelle
A, $R_1$, $R_2$ et n ont les significations mentionnées ci-dessus,
ou bien on transforme ultérieurement les composés de formule générale IV ainsi obtenus en d'autres composés de formule générale IV.

**15.** Composés de formule générale VII

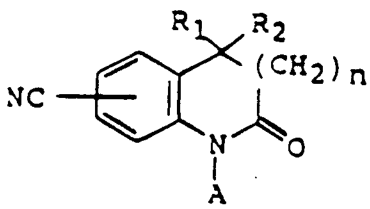

(VII)

dans laquelle

- A représente un atome d'hydrogène, un groupe alkyle($C_1$-$C_6$), alcényle($C_2$-$C_6$), alcynyle($C_2$-$C_6$), benzyle ou cycloalkyle($C_3$-$C_7$),
- $R_1$ représente un atome d'hydrogène, un groupe alkyle($C_1$-$C_6$). alcényle($C_2$-$C_6$) ou cycloalkyle-($C_3$-$C_7$),
- $R_2$ représente un atome d'hydrogène, un groupe alkyle($C_1$-$C_4$), alcényle($C_2$-$C_6$), cycloalkyle($C_3$-$C_7$), alkyl($C_1$-$C_6$)-carbonyle, alcoxy($C_1$-$C_6$)-carbonyle, aminocarbonyle ou hydrazinocarbonyle, ou bien $R_1$ et $R_2$ forment, conjointement avec l'atome de C auquel ils sont liés, un cycle cycloalkyle($C_3$-$C_7$), et
- n peut prendre la valeur de 0 ou de 1,

à l'exception du composé 2,3-dihydro-2-oxo-(1H)-indol-5-carbonitrile.

**16.** Procédé de préparation des composés de formule générale VII selon la revendication 15, caractérisé en ce qu'on diazote des composés de formule générale (VIII)

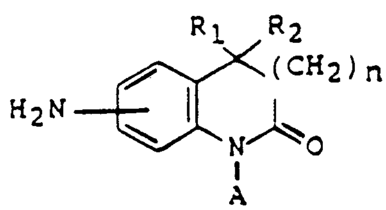

(VIII)

dans laquelle A, $R_1$, $R_2$ et n ont les significations mentionnées ci-dessus, et on les fait réagir avec des cyanures (réaction de Sandmeyer).

**17.** Utilisation des composés selon la revendication 13 ou 15 pour la préparation de composés selon l'une quelconque des revendications 1-8.